# EUROPEAN PATENT SPECIFICATION

(11) **EP 2 914 308 B1**
(45) Date of publication and mention of the grant of the patent: **06.12.2017**
(21) Application number: 13792132.6
(22) Date of filing: 30.10.2013
(51) Int. Cl.: A61L 27/36, A61L 27/38, A61L 27/50, A61L 27/54

(54) **REINFORCED TISSUE GRAFT**
VERSTÄRKTES GEWEBETRANSPLANTAT
GREFFON TISSULAIRE RENFORCÉ

(30) Priority: 30.10.2012 US 201213663938
(43) Date of publication of application: 09.09.2015
(73) Proprietor: THE CLEVELAND CLINIC FOUNDATION, Cleveland, OH 44195 (US)
(72) Inventor: DERWIN, Kathleen, A., Cleveland, OH 44195 (US); IANNOTTI, Joseph, P., Cleveland, OH 44195 (US); SAHOO, Sambit, Cleveland, OH 44195 (US)
(74) Representative: FRKelly
(86) International application number: PCT/US2013/067514
(87) International publication number: WO 2014/070896

(56) References cited:
- EP-A1- 1 177 800
- WO-A2-2007/047743
- WO-A2-2009/120966

## Description

### Technical Field

The present invention is directed to tissue grafts and, in particular, is directed to a reinforced tissue graft.

### Background of the Invention

Current treatment for rotator-cuff tears is to suture the torn tendon back to the bone of the humeral head. The sutures hold the tendon in contact with the bone, preferably long enough for the tendon to heal to the bone and form a bridge that will re-establish the tendon-bone connection and restore normal function. The sutures that are used possess sufficient tensile strength to retain the tendon and bone together during the healing process. However, the tendon is a fibrous tissue that can be torn by the sutures. The sutures can align with the fascicular structure of the tendon and tear through it under sufficient tensile force undoing the surgical repair before tendon-to-bone healing is complete. The sutures can also tear through the bone under sufficient force, particularly in older subjects who form the bulk of rotator-cuff-tear patients and whose bones tend to be more osteoporotic.

WO2009120966 concerns a biocompatible tissue graft including an extracellular matrix patch and a means for reinforcing the patch. EP1177800 concerns a bioprosthetic device [10] for soft tissue attachment, reinforcement, and or reconstruction, in which the device comprises a naturally occurring extracellular matrix portion [12] and a synthetic portion [14]. WO2007047743 concerns a medical device with pre-attached affixation means placed to minimize the likelihood of adjacent tissues undesirably adhering to the affixation means.

### Summary of the Invention

The present invention relates to a reinforced, biocompatible tissue graft. The tissue graft includes an extracellular matrix patch (ECM) that has a peripheral surface and a means for reinforcing the graft to mitigate tearing of the graft, improve the fixation retention of the graft, and/or limit cyclic stretching of the graft and substantially maintain these properties following partial enzymatic degradation of the graft while it remodels. The reinforcing means includes at least one free end that extends beyond the peripheral surface for securing the patch to a host tissue. The reinforcing means can include a fiber stitched into the ECM patch in a reinforcement pattern. The fiber can be formed from a biocompatible material and have a high modulus of elasticity and failure load. Examples of biocompatible materials that can be used to form the fiber include silk, sericin-free silk, modified silk fibroin, polyesters, such as poly(glycolic acid) (PGA), poly(lactic acid) (PLA), poly(ethylene glycol) (PEG), polyhydroxyalkanoates (PHA) and polyethylene terephthalate (PET), medical grade polyethylene, such as polyethylene (UHMWPE), blends thereof and copolymers thereof, as well as other biocompatible materials that are typically used in forming biocompatible fibers for *in vivo* medical applications. The fiber can additionally be radio-opaque (*e.g.*, by adding an opacifier, such as barium sulfate or tantalum to the fiber).

Another aspect of the present invention relates to a method of constructing a biocompatible tissue graft that includes providing an extracellular matrix patch and stitching at least one fiber into the patch in a reinforcement pattern to mitigate tearing of the graft, improve the fixation retention of the graft, and/or limit cyclic stretching of the graft and substantially maintain these properties following partial enzymatic degradation of the graft while it remodels. The at least one fiber can have a free end that extends beyond the peripheral surface. The free end of the at least one fiber is secured to a host tissue.

Still a further aspect of the present invention relates to a tissue graft for use in a method for repairing tissue in a subject that includes providing a tissue graft having a peripheral surface and including an extracellular matrix patch and at least one fiber stitched into the patch to mitigate tearing of the graft, improve the fixation retention of the graft, and/or limit cyclic stretching of the graft and substantially maintain these properties following partial enzymatic degradation of the graft while it remodels. A free end of the at least one fiber extends beyond the peripheral surface of the tissue graft and is secured to the tissue in the subject.

### Brief Description of the Drawings

The foregoing and other features and advantages of the present invention will become apparent to those skilled in the art to which the present invention relates upon reading the following description with reference to the accompanying drawings, in which:
Fig. 1 is schematic illustration of a tissue graft having reinforcement means in accordance with an embodiment of the present invention;
Fig. 2 is a top view of a tissue graft having a reinforcement means in accordance with another embodiment of the present invention;
Fig. 3a is a top view of a tissue graft having a reinforcement means in accordance with yet another embodiment of the present invention;
Fig. 3B is a top view of a tissue graft having a reinforcement means in accordance with yet another embodiment of the present invention;
Fig. 3C is a top view of a tissue graft having a reinforcement means in accordance with yet another embodiment of the present invention;
Fig. 4A is a schematic illustration of a tissue graft having a reinforcement means in accordance with yet another embodiment of the present invention;
Fig. 4B is a top view of a tissue graft having a reinforcement means in accordance with yet another embodiment of the present invention;
Fig. 5A is a schematic illustration of a fiber of a tissue graft in accordance with yet another embodiment of the present invention;
Fig. 5B is a graph illustrating the uniaxial suture retention strength of unreinforced and reinforced tissue grafts;
Fig. 6 is a graph illustrating the multi-directional suture retention strength of unreinforced and reinforced tissue grafts;
Fig. 7 is a graph illustrating the multi-directional suture retention strength of unreinforced and reinforced tissue grafts before and after 21 days incubation in 1x PBS (pH=7.4) at 37°C;
Fig. 8 is a graph illustrating load-displacement plots of unreinforced tissue grafts and tissue grafts reinforced using different stitch designs and tested using a multi-directional ball burst test;
Fig. 9 is a graph illustrating load displacement plots of unreinforced tissue grafts and tissue grafts reinforced using different stitch designs tested in uniaxial tension with side constraint;
Fig. 10 is a graph illustrating the uniaxial suture retention strength of unreinforced and reinforced tissue grafts using a peripheral stitch design;
Fig. 11 is a graph illustrating the multi-directional suture retention strength of unreinforced and reinforced tissue grafts using a peripheral stitch design;
Fig. 12 is a graph illustrating the uniaxial suture retention strength of unreinforced and reinforced tissue grafts using a rectangular cross-hatch stitch design;
Fig. 13 is a graph illustrating the cyclic elongation during uniaxial fatigue loading of unreinforced and reinforced tissue grafts using a rectangular cross-hatch stitch design;
Fig. 14 is a schematic illustration of bone fixation methods for unreinforced and reinforced grafts;
Fig. 15 is a graph illustrating the failure load of unreinforced and reinforced tissue grafts for various bone fixation methods;
Fig. 16 is a graph illustrating the cyclic creep of unreinforced and reinforced tissue grafts for various bone fixation methods;
Fig. 17 is a graph illustrating the uniaxial load-displacement curve of reinforced tissue grafts for various bone fixation methods;
Fig. 18 is a graph illustrating the uniaxial suture retention strength of unreinforced tissue grafts and tissue grafts reinforced with resorbable and non-resorbable fibers;
Fig. 19 is a graph illustrating the uniaxial load-displacement curves of tissue grafts reinforced with resorbable and non-resorbable fibers;
Fig. 20A is a schematic illustration of a tissue graft having a reinforcement means that extends beyond the periphery of the tissue graft in accordance with an aspect of the present invention;
Fig. 20B is a schematic illustration of a tissue graft having a reinforcement means that extends beyond the periphery of the tissue graft in accordance with another aspect of the present invention;
Fig. 21A is a schematic illustration of a tissue graft having a reinforcement means that extends beyond the periphery of the tissue graft in accordance with yet another aspect of the present invention;
Fig. 21B is a schematic illustration of a tissue graft having a reinforcement means that extends beyond the periphery of the tissue graft in accordance with yet another aspect of the present invention;
Fig. 22 is a schematic illustration of a tissue graft having a reinforcement means that extends beyond the periphery of the tissue graft in accordance with yet another aspect of the present invention;
Fig. 23 is a schematic illustration of a tissue graft having a reinforcement means that extends beyond the periphery of the tissue graft in accordance with yet another aspect of the present invention;
Fig. 24 is a schematic illustration of a circular tissue graft having a reinforcement means that extends beyond the periphery of the tissue graft in accordance with yet another aspect of the present invention;
Fig. 25A is a schematic illustration of a tissue graft having reinforcement means that includes concentric and linearly extending portions with free ends that extend beyond the periphery of the tissue graft in accordance with yet another aspect of the present invention;
Fig. 25B is a schematic illustration of another tissue graft having reinforcement means that includes concentric and linearly extending portions with free ends that extend beyond the periphery of the tissue graft in accordance with yet another aspect of the present invention;
Fig. 26 is a photograph illustrating the tissue graft of Fig. 20A;
Fig. 27A is a photograph illustrating a rotator cuff repair augmented with a reinforced tissue graft having a reinforcement means that extends beyond the periphery of the tissue graft;
Fig. 27B is a photograph illustrating a rotator cuff repair augmented with only a reinforcing fiber;
Fig. 28 is a graph illustrating the cyclic gap formation of rotator cuff repairs without augmentation or augmented with a reinforced tissue graft or a reinforcing fiber alone;
Fig. 29 is a graph illustrating the cyclic gap formation of rotator cuff repairs without augmentation or augmented with additional reinforced tissue grafts or reinforcing fibers alone; and
Fig. 30 is a graph illustrating cumulative cyclic elongation (CCE) curves of native and fiber-reinforced dermis patches before and after enzymatic degradation.

### Detailed Description

The present invention is directed to tissue grafts and, in particular, is directed to a fiber reinforced tissue graft with improved fixation retention, suture retention, and biomechanical strength properties. The tissue graft can be used to treat a tissue defect of a subject (*e.g.*, human being), such as a musculoskeletal defect, or in tendon-to-bone repairs (*e.g*., rotator cuff injury), or soft-tissue repairs, such as the repair of lacerated muscles, muscle transfers, or use in tendon reinforcement. The tissue graft may also be used as a bridging material in a subject in the case where the gap between a tendon and the associated bone is too large to repair conventionally. The tissue graft can be incorporated between the bone-tendon interface and fixed to the bone and tendon to repair a gap or tear.

The tissue graft in accordance with the present invention includes an extracellular matrix (ECM) patch (or ECM) and a reinforcing means. The ECM can be derived from any mammalian ECM, such as fascia, and in particular, fascia lata from humans. The ECM can be derived from other connective tissue materials, such as dermis as long as the ECM is biocompatible with the target site or the tissue injury being treated in the subject or both. The ECM can also be derived, for example, from other tissues and/or other materials, such as collagen, skin, bone, articular cartilage, meniscus, myocardium, periosteum, artery, vein, stomach, large intestine, small intestine, diaphragm, tendon, ligament, neural tissue, striated muscle, smooth muscle, bladder, ureter, abdominal wall fascia, and combinations thereof.

The ECM used to form the tissue graft may be obtained directly from mammalian tissue (such as an autograft, allograft or xenograft). These tissues may be obtained from patients at the time of surgery or a commercial source, such as a tissue bank medical device company. ECM obtained from tissue banks and other commercial sources may be formed using proprietary processing techniques or modified by additional processing techniques before it is used. In one example, these techniques can be used to remove cells and other potentially infectious agents from the ECM.

The reinforcing means can include any structure or material that is applied to the ECM, is capable of mitigating tearing of the graft when the graft is fixed to tissue being treated, is capable of increasing or improving the fixation retention properties of the tissue graft beyond that which is present in a patch of the ECM alone, and/or can limit the cyclic stretching of the graft and substantially maintain these properties following partial enzymatic degradation of the graft while it remodels. The fixation retention properties can be tailored to increase the graft's ability to remain secured to anatomic structures, such as bone and soft tissues, when used to treat a tissue defect. The graft may be secured to these anatomical structures by, for example, weaving, screws, staples, sutures, pins, rods, other mechanical or chemical fastening means or combinations thereof. For instance, the graft may be secured to the treated tissue via different suture configurations, such as, massive cuff, mattress stitching and simple suture and different fixation techniques, such as, Synthes screw or Biotenodesis screw fixation and suture anchors with a Krakow stitch.

In one aspect of the invention, the reinforcing means can include a thread or strands of fiber(s) that are stitched in a reinforcement pattern in the ECM patch. Fiber stitched in a reinforcement pattern can increase the fixation properties of the tissue graft, which will result in a tissue graft having improved mechanical properties for implantation and repair of anatomical defects in a subject. The reinforcement pattern can include any stitch pattern that mitigates tearing of the graft, improves the fixation retention of the graft, and/or limits cyclic stretching of the graft and substantially maintain these properties following partial enzymatic degradation of the graft while it remodels. For example, the stitch pattern can include one or more generally concentric, peripheral or cross-hatched stitch patterns.

The fiber can enhance the fixation retention, mitigate tearing, and limit cyclic stretching of the tissue graft once stitched into the graft. The fiber can be formed from a biocompatible material that is bioresorbable, biodegradable, or non-resorbable. The term bioresorbable is used herein to mean that the material degrades into components, which may be resorbed by the body and which may be further biodegradable. Biodegradable materials are capable of being degraded by active biological processes, such as enzymatic cleavage.

One example of a biocompatible material that can be used to form the fiber is silk. The silk may include, for example, sericin-free silk fibroin or silk-fibroin modified with a peptide sequence that sequesters growth factors *in vivo,* such as disclosed in U.S. Patent No. 6,902,932, which is herein incorporated by reference. The fibers can also be formed from biodegradable polymers including poly(glycolic acid) (PGA), poly(lactic acid) (PLA), poly(lactic-co-glycolic acid) (PLGA), poly(ethylene glycol) (PEG), blends thereof, and copolymers thereof. By way of example, the reinforcing fiber may include a core of PGA surrounded by a sheath of reinforced PLA fibers. The PGA and PLA may be obtained, for example, from Concordia Fibers in Coventry, RI. Other examples of biocompatible polymers that can be used to form the fiber are resorbable polyesters, such as polyhydroxyalkanoates (PHA), and non-resorbable fibers, such as polyethylene terephthalate (PET) and ultra-high molecular weight polyethylene (UHMWPE). It will be appreciated that the biocompatible fiber can be formed from other biocompatible materials, such as other biocompatible materials that are typically used in forming biocompatible fibers for *in vivo* medical applications.

In another example, the biocompatible polymer used to form the fibers may be radio-opaque to allow the location, integrity, and/or deformation (e.g., contraction or distension) of the tissue graft to be assessed in a living system. Such a radio-opaque fiber will also mitigate tearing of the graft, improve the fixation retention of the graft, and/or limit cyclic stretching of the graft and substantially maintain these properties following partial enzymatic degradation of the graft while it remodels.

Regardless of the material used for the fiber of the reinforcing means, the fiber should exhibit a high modulus of elasticity and a failure load tailored to meet particular design criterion corresponding with *in vivo* strength requirements of the treated tissue. For example, reinforced patches used for the treatment of large and massive rotator cuffs should exhibit failure loads of greater than about 250 Newtons (N) at a time of implantation, and greater than about150 N after about one week of implantation *in vivo.* Alternatively, reinforcing patches used for the treatment of tissues experiencing lower natural loads may be required to exhibit failure loads of about 30 N to about 50 N. It will be understood, however, that the fibers, their stitch design (*i.e.,* reinforcement pattern), or the particular ECM can be tailored to produce failure loads of the fiber-reinforced ECM patch commensurate in scale to any tissue treated within the body.

In an aspect of the invention, the fibers and/or the ECM can be mechanically, chemically or biologically modified to enhance adhesion between the fibers and ECM to further secure the fibers to the ECM. This modification may occur before or after the fibers are incorporated into the ECM. This modification may be performed on a portion of or substantially all of the stitched fibers or the ECM or both. During loading of the tissue graft, the fibers may begin to displace relative to the ECM and may ultimately completely slip out from the ECM and become the primary load bearing components of the reinforced tissue construct. It therefore becomes desirable to mitigate or prevent fiber slippage in order to ensure that usage loads are borne by the entire graft and not just the fibers. Adhesion characteristics of the fibers can be improved by ablation via ultra-violet (UV) or infrared (IR) light, UV cross-linking or chemical cross-linking, plasma etching, ion etching, coating the fibers with microspheres, application of bioadhesives or combinations thereof. These treatments can likewise be performed on the ECM.

In another aspect of the invention, the ECM can be processed to become decellularized. Once decellularized, cells can be seeded into the decellularized ECM that enhance the therapeutic potential of the tissue graft. For example, the ECM can be seeded with a plurality of progenitor cells that become dispersed in the ECM. Examples of progenitor cells are known in the art and can include bone marrow-derived progenitor cells, hematopoietic stem cells, endothelial progenitor cells, mesenchymal stem cells, multipotent adult progenitor cells (MAPCs), embryonic stem cells, stromal cells, stem cells, embryonic stem cells, chondrocytes, osteoblasts, and tenocytes. The progenitor cells can be autologous, allogeneic, xenogeneic or a combination thereof. The progenitor cells can also be genetically modified. Genetically modified cells can include cells that are transfected with an exogenous nucleic acid and that express a polypeptide of interest including, for example, a growth factor, a transcription factor, a cytokine, and/or a recombinant protein.

The ECM can additionally or optionally include at least one biologically active molecule dispersed or seeded therein. Any desired biologically active molecule can be selected for impregnating into the ECM. For example, the biologically active molecule can include enzymes, hormones, cytokines, colony-stimulating factors, vaccine antigens, antibodies, clotting factors, angiogenesis factors, regulatory proteins, transcription factors, receptors, and structural proteins. The biologically active molecule can be chosen based on where the musculoskeletal graft is to be located in the subject or the physiological requirements of the subject or both. For example, if the musculoskeletal graft is used to repair a tendon, the biologically active molecule which is seeded on or into the ECM can be a growth factor such as IGF-I, TGF-β, VEGF, bFGF, BMP or combinations thereof.

Optionally, a high-molecular weight (*e.g*., greater than about 250 kDa) hyaluronic acid (HA) can be incorporated into the tissue graft prior to, during, or after stitching of the fibers into the ECM. When incorporated into the tissue graft, HA can potentially inhibit the migration of inflammatory cells, induce the migration of non-inflammatory cells, and promote angiogenesis, which would promote integration of the ECM with the underlying host tissues.

The high-molecular weight HA can be cross-linked within the ECM to mitigate diffusion of the HA from the ECM. Cross-linked, high-molecular-weight HA can be retained in ECM for extended periods *in vitro.* An example of a cross-linked HA material that can be used in this application is prepared by substituting tyramine moieties onto the HA chains and then linking tyramines to form dityramine linkages between HA chains, effectively cross-linking or gelling the HA into the ECM. Examples of dityramine-cross-linked HA composition and chemistry are disclosed in U.S. Patent Nos. 6,982,298 and 7,465,766 and U.S. Patent Application Publications Nos. 2004/0147673 and 2005/0265959. The tyramine-substitution rate on the HA molecules may be about five percent based on available substitution sites as disclosed in the aforementioned publications.

TS-HA can be impregnated into the ECM, and then immobilized within ECM by cross-linking of the tyramine adducts to form dityramine linkages, thereby producing a cross-linked HA macromolecular network. The TS-HA can be impregnated into the ECM prior to or after stitching the ECM.

The TS-HA can be used to attach fibronectin functional domains (FNfds) to the ECM in order to further promote healing, cell migration, and anti-inflammatory capabilities. FNfds possess the ability to bind essential growth factors that influence cell recruitment and proliferation (*e.g*., PDGF-BB and bFGF). The FNfds may, for example, constitute fibronectin peptide "P-12" with a C-terminal tyrosine to allow it to be cross-linked to TS-HA.

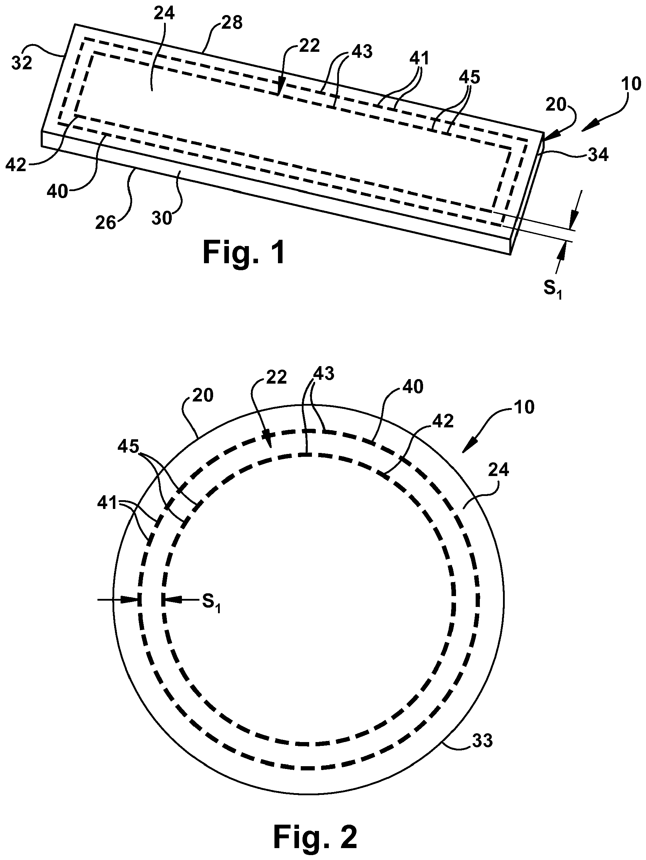

One example of a tissue graft in accordance with the present invention is illustrated in Fig. 1. The tissue graft 10 includes a reinforced ECM patch or strip that can be used to augment a tendon or muscle repair to bone in, for example, a rotator cuff injury. The tissue graft 10 includes an ECM patch 20 and a means 22 for reinforcing the patch.

The patch 20 is illustrated as having a generally rectangular strip shape (*e.g*., about 5 cm long by about 2 cm wide) although the patch can have other shapes, such as an elliptical shape, a circular shape, a square shape, etc. (*e.g*., Figs 2, 3, 4). The patch 20 includes a top surface 24 and a substantially parallel bottom surface 26 spaced from the top surface. A first side 28 and second side 30 connect the top surface 24 to the bottom surface 26. The first and second sides 28, 30 extend generally parallel to one another. The patch 20 further includes a front surface 32 and rear surface 34 which connect the first side 28 to the second side 30. The front and rear surfaces 32, 34 extend generally parallel to one another.

The reinforcing means 22 can include at least one fiber disposed or provided within the patch 20 by, for example, conventional stitching techniques. By stitching, it is meant that at least one fiber of the reinforcing means 22 is stitched into the patch 20 such that each stitch of the reinforcing means extends between and through both the top surface 24 and the bottom surface 26 of the patch 20 to securely fasten the reinforcing means to the patch.

The reinforcing means 22 may exhibit any reinforcement configuration or pattern that increases the fixation properties of the patch 20. One such configuration is illustrated in Fig. 1 in which first and second fibers 40, 42 are stitched into the patch 20 in geometrically concentric configurations. Additionally or alternatively, the stitch lines of the fibers can be placed further away from the edges of the patch 20 to delay, mitigate, or prevent slipping of the fibers 40, 42 within the patch 20. Although Fig. 1 illustrates two fibers in a geometrically concentric pattern, it will be understood that more or fewer fibers can be stitched into the patch in a geometrically concentric pattern. Additionally, it will be appreciated that additional fibers can be stitched into the ECM patch 20 in other reinforcement patterns.

As shown in Fig. 1, the first fiber 40 can extend substantially parallel to, and be spaced inwardly from, the periphery of the patch 20. By way of example, the first fiber 40 can extend substantially parallel to the first and second sides 28, 30 and the front and rear surfaces 32, 34 of the patch 20 such that the first fiber 40 exhibits a generally rectangular configuration. The first fiber 40 can comprise a plurality of interconnected stitches 41.

The second fiber 42 can extend substantially parallel to the first fiber 40 and be disposed radially inward of the first fiber 40 within the patch 20. In this configuration, the first and second fibers 40, 42 form a generally geometrically concentric construction in a peripheral double pass orientation. The second fiber 42 can comprise a plurality of interconnected stitches 45 The second fiber can be substantially uniformly spaced inward from the first fiber 40 by a gap indicated by "s₁". The gap s₁ may be, for example, on the order of about 1 mm to about 3 mm (*e.g*., about 2 mm), although other spacing configurations will be understood. It will be appreciated that although the gap s₁ between the fibers 40 and 42 is substantially uniform, the gap s₁ may vary depending on reinforcement pattern in which the fibers 40 and 42 are stitched.

The first fiber 40 and the second fiber 42 can be stitched in the ECM so that the number of stitches per inch is, for example, about 10 stitches per inch to about 20 stitches per inch (*e.g*., about 15 stitches per inch). Generally, the more stitches per inch, the greater the strength of the reinforcing means 22 and the fixation retention properties of the tissue graft 10. In some examples, however, it may be desirable to use less stitches per inch to avoid excessive needle penetrations in the ECM 20, which may potentially weaken the tissue graft 10.

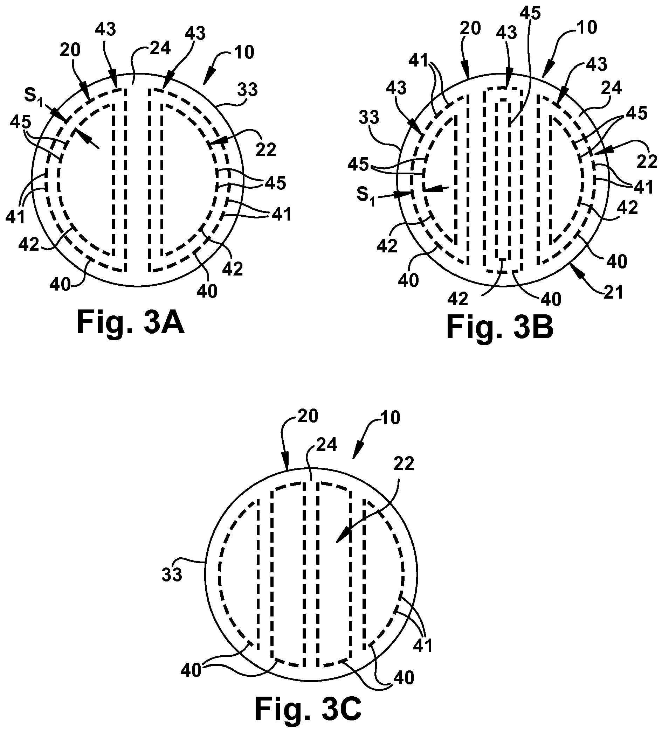

Other examples of concentric reinforcement stitch patterns or configurations are illustrated in Fig. 2 and Figs. 3A-3C. The configurations in Fig. 2 and Figs. 3A-3C are similar to the configuration of Fig. 1, except that in Fig. 2 the patch 20 is substantially circular and therefore the reinforcing means 22 is provided in the patch in a generally circular configuration or orientation. Fig. 2 illustrates one example of a patch 20 that includes generally concentric reinforcement means 22 in a peripheral double pass orientation. The reinforcement means 22 includes a first fiber 40 that comprises a plurality of interconnected stitches 41 and a second fiber 42 that comprises a plurality of interconnected stitches 45. The first fiber 40 can extend substantially parallel to, and be spaced inwardly from, a peripheral surface 33 of the patch 20 such that the first fiber has a generally circular configuration. The second fiber 42 can extend substantially parallel to the first fiber 40 and be disposed radially inward of the first fiber within the patch 20. In this configuration, the first and second fibers 40, 42 form a generally concentric construction.

Fig. 3A illustrates another example of the reinforcing means 22 comprising two concentric patterns 43. Each concentric pattern 43 includes a first strand 40 that comprises a plurality of interconnected stitches 41 and a second strand 42 that comprises a plurality of interconnected stitches 45. Each first fiber 40 can extend substantially parallel to, and be spaced inwardly from, a peripheral surface 33 of the patch 20 such that each first fiber has a generally circular configuration. Each second fiber 42 can extend substantially parallel to the first fiber 40 and be disposed radially inward of the first fiber within the patch 20. In this configuration, each pair of first and second fibers 40, 42 form a generally concentric construction. Although the two concentric patterns 43 are illustrated as being substantially semi-circular, it will be understood that each concentric pattern may exhibit alternative constructions such as, for example, rectangular (*e.g*., in a two rectangle double pass orientation), elliptical, triangular or combinations thereof.

Fig. 3B illustrates another example of the reinforcing means 22 comprising three concentric patterns 43. Each concentric pattern 43 comprises a first strand 40 comprising a plurality of interconnected stitches 41 and a second strand 42 comprising a plurality of interconnected stitches 43. Each first fiber 40 can extend substantially parallel to, and be spaced inwardly from, a peripheral surface 33 of the patch 20 such that each first fiber has a generally circular configuration. Each second fiber 42 can extend substantially parallel to the first fiber 40 and be disposed radially inward of the first fiber within the patch 20. In this configuration, each pair of first and second fibers 40, 42 form a generally concentric construction. It will be understood that each concentric pattern may exhibit any constructions such as, for example, rectangular (*e.g*., in a three rectangle double pass orientation), elliptical, triangular, semi-circular, circular or combinations thereof.

Fig. 3C illustrates yet another example of a reinforcing means reinforcing means 22 that includes a plurality of first strands 40, which comprise a plurality of interconnected stitches 41 but without or free of concentric second strands 42. In particular, the first strands 40 may comprise four substantially parallel and elliptical discrete first strands. Although the four first strands 40 are illustrated as being substantially elliptical, it will be understood that each first strands may exhibit alternative constructions such as, for example, rectangular (*e.g*., in a four rectangle single pass orientation), semi-circular, circular, triangular or combinations thereof within the spirit of the present invention. It will also be understood that one or more of the first strands could have a geometrically concentric pattern with a second strand within the spirit of the present invention.

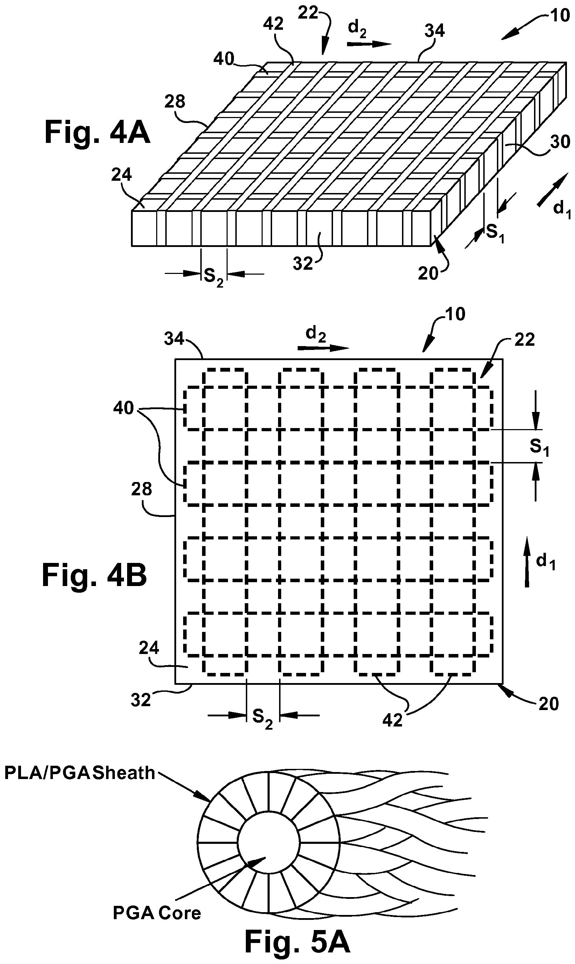

Fig. 4A is a schematic illustration of a tissue graft 10 that includes an ECM patch 20 and a reinforcing means 22 in accordance with another example of the invention. The reinforcing means 22 includes a plurality of first fibers 40 and a plurality of second fibers 42 stitched in a cross-hatched pattern across the patch 20 and between the first and second sides 28, 30 and the front and rear surfaces 32, 34. Although Fig. 4A illustrates six first fibers 40 and eight second fibers 42, it is understood that more or less of each fiber may be utilized in accordance with the present invention. The first fibers 40 can extend in a first direction, indicated by "d₁", across the top surface 24 of the patch 20 from the first side 28 to the second side 30. Each of the first fibers 40 can extend parallel to one another and be spaced apart by a gap indicated by "s₁". The gap s₁ may be, for example, on the order of about 1 mm to about 3 mm, although other spacing configurations will be understood. The gap s₁ may be uniform or may vary between first fibers 40.

The second fibers 42 can extend in a second direction, indicated by "d₂", across the top surface 24 of the patch 20 from the front surface 32 to the rear surface 34. The directions "d₁" and "d₂" in which the first and second fibers 40, 42 extend may be configured such that the first fibers and the second fibers are oriented perpendicular to each other. Each of the second fibers 42 can extend parallel to one another and be spaced apart by a gap indicated by "s₂". The gap s₂ may be, for example, on the order of about 1 mm to about 3 mm, although the gap can have other spacing configurations. The gap s₂ may be uniform or may vary between second fibers 42. The second fibers 42 are disposed in an overlying fashion relative to the first fibers 40 such that the first fibers are disposed between the top surface 24 of the patch 20 and the second fibers. The second fibers 42, however, could alternatively be disposed between the top surface 24 of the patch 20 and the first fibers 40.

Fig. 4B illustrates that the reinforcing means 22 comprises a plurality of first fibers 40 and a plurality of second fibers 42 stitched in a cross-hatched pattern across the patch 20 and between the first and second sides 28, 30 and the front and rear surface 32, 34. Although Fig. 4B illustrates four first fibers 40 and four second fibers 42, it is understood that more or less of each fiber may be utilized in accordance with the present invention. Each of the first fibers 40 extends from the first side 28 to the second side 30 of the patch 20. Each of the second fibers 42 extends from the front surface 32 to the rear surface 34 of the patch 20.

The second fibers 42 are disposed in an overlying fashion relative to the first fibers 40 such that the first fibers are disposed between the top surface 24 of the patch 20 and the second fibers. The second fibers 42, however, could alternatively be disposed between the top surface 24 of the patch 20 and the first fibers 40. Although the first and second fibers 40, 42 are illustrated as having a substantially rectangular shape (*e.g*., a rectangular cross-hatch orientation), it will be understood that the first fiber 40 and/or the second fiber 42 may exhibit alternative constructions such as elliptical, semi-circular, circular, triangular or combinations thereof.

The tissue graft of the present invention can be used in tissue engineering and musculoskeletal repair, such as rotator cuff repair, but is not restricted to musculoskeletal applications. The graft may be administered to a subject to mechanically and biologically augment the repair by placing it over a tendon-bone repair or interpositionally grafting a rotator cuff tendon defect. It will be appreciated that similar methods and materials as described herein could also be adapted to other tendon-to-bone repairs, soft-tissue repairs, such as the repair of lacerated muscles, muscle transfers, spanning a large muscle defect, or use in tendon reinforcement. These applications require secure connections between the graft 10 and the anatomical site. Fixation techniques to soft tissue using conventional or novel suture methods, or the Pulvertaft weave technique (M. Post, J Shoulder Elbow Surg 1995; 4:1-9) may be utilized in accordance with the present invention. Fixation techniques to bone using conventional or novel suture methods, anchors, screws, or plates may be utilized in accordance with the present invention.

The graft 10 may also serve as a delivery platform for the future investigation of any number of biologic strategies aimed to enhance muscular skeletal repair, *e.g.*, rotator cuff healing. Furthermore, the graft 10 could be effective for other needs in the field of surgical reconstruction, including ligament reconstruction, bowel and bladder reconstruction, abdominal wall repair, and tendon reconstruction in the setting of post-surgical repair failure, trauma and segmental defects. The following examples are illustrative of the principles and practice of this invention.

### Example 1

In this example, two groups were investigated: Group I-Control - Native (unreinforced) fascia and Group II-Experimental - reinforced fascia. The fascia was reinforced using a biodegradable polymer braids as the reinforcing material. Polymer braids used were made of poly lactic acid (PLA) and poly glycolic acid (PGA). PLA and PGA are the most widely researched polymers in the field of tissue engineering. Since PGA degrades more rapidly than PLA, the present example uses polymer braids having PGA as a core and a combination of PLA/PGA as a sheath (Fig. 5A). Two tests were used to verify the efficacy of stitching as a method of reinforcement with polymer braids, namely, uniaxial tension test and multi directional loading using a modified Ball burst test.

All allograft human fascia lata were obtained from the Musculoskeletal Transplant Foundation in Edison, NJ (donor age 18-55 years). All PGA and PLA braids used for reinforcing the fascia were obtained from Concordia Fibers, Coventry, RI.

### Uniaxial suture retention test

A sample size of (n=10) unreinforced patches were used as the control. Each consisted of 2 cm wide x 5 cm long strips of ECM hydrated for 20 minutes in saline solution and maintained at room temperature. The unreinforced patches were tested with either one mattress (n=5) or one Mason Allen suture (n=5) placed 5 mm away from the 2 cm wide edge. A template was created to assure uniformity in the placement of the sutures. The two sutures were tied over a tubular rod mounted on the cross head of a MTS 5543 table top system.

A sample size of (n=7) reinforced fascia were used to test the ability of stitching with two types of PLA/PGA braid to improve the suture retention strength over unreinforced fascia. This sample size was selected due to the preliminary nature of the study. Each reinforced patch consisted of fascia 2 cm wide x 5 cm long reinforced with a polymer braid having a sheath of 4PLA and 4PGA with (n=2) and without (n=5) a 2PGA core. The reinforced patches were tested with two simple sutures placed 5 mm away from the 2 cm wide edge. The suture retention load was defined as the maximum load attained by the specimen.

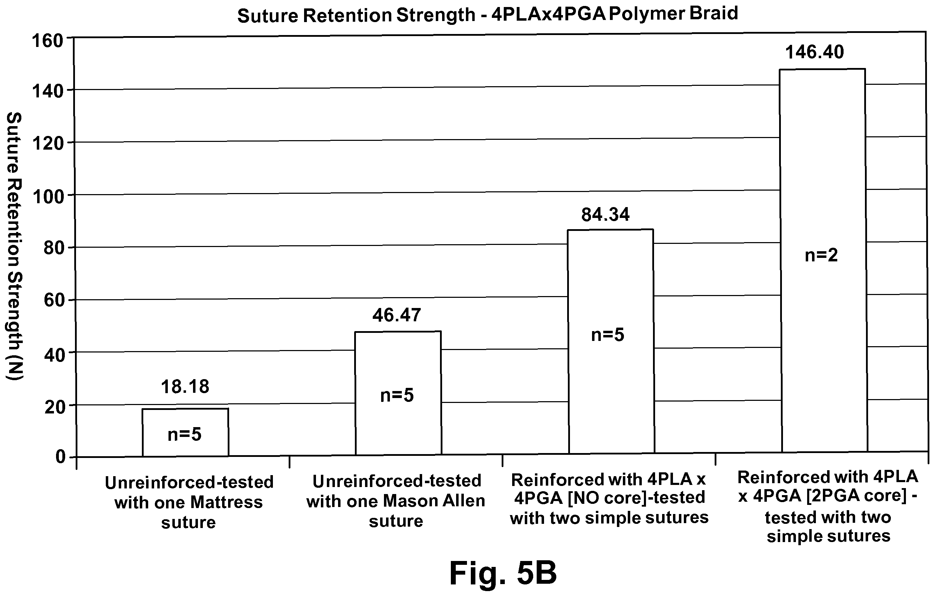

The results of this test are illustrated in Fig. 5b. Fig. 5b shows that stitching as a reinforcement method has the ability to increase the suture retention strength of fascia. Additionally, the presence of a PGA core in the polymer braid positively impacts the reinforcement by increasing the suture retention strength of the tissue. The suture retention loads obtained with the polymer braids having no PGA core (84 N) were about half the suture retention loads attained with polymer braids having a PGA core (146 N).

### Ball Burst test

Depending on the size, location, and chronicity of the tear *in vivo,* the graft may be subjected to biaxial tensile forces. Therefore, experiments using a modified Ball Burst test, inspired from the ASTM D3787 Ball Burst test standard used to determine the bursting resistance of knitted fabrics and goods under multi-axial forces, were used to quantify the suture retention strength. In particular, 4 cm diameter discs of the unreinforced and reinforced fascia were hydrated for 20 minutes in saline at room temperature. The hydrated specimens were then sutured to a stainless steel ring (5 cm outer diameter) in a simple suture configuration at 1 cm increments using No. 2 Fiberwire (Arthrex Corporation, Naples, FL). The fasciasteel construct was then mounted on a specially designed fixture, which was then mounted on the base of the MTS 1321 system. A polished stainless steel ball having a 1" diameter was attached to the cross head of the MTS system and pushed through the specimen at a constant distraction rate of 6 mm/min. The suture retention load was noted as the maximum load attained by the specimen prior to a 10% or more drop in the peak load.

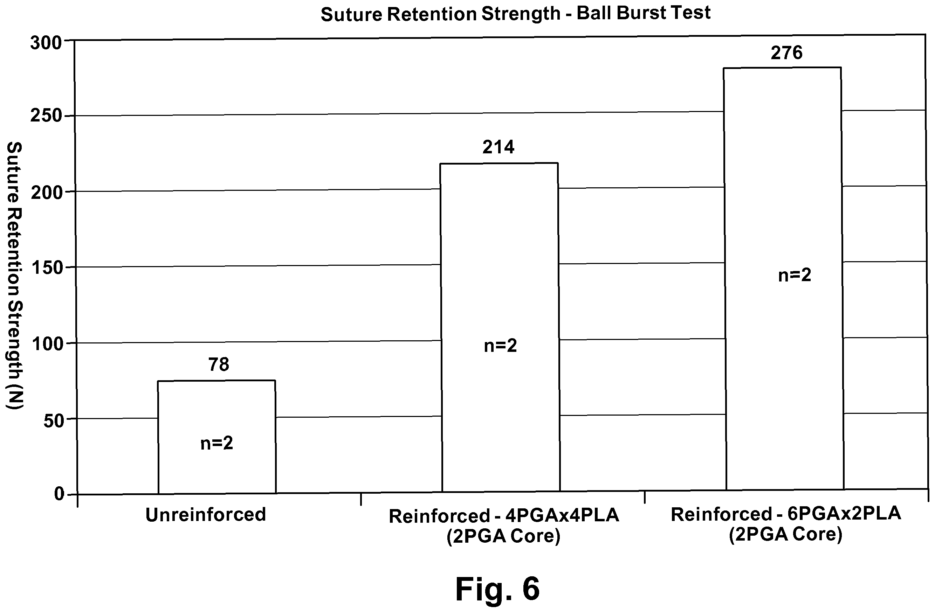

The results of the Ball Burst test are illustrated in Fig. 6. Fig. 6 shows that even with the Ball Burst test, which is more rigorous than the uniaxial test in that it subjects the specimen to multi-directional loading, the reinforced fascia construct has suture retention strength about 3 to about 4 times greater than unreinforced fascia. Fig. 6 also illustrates that the amount of PGA in the polymer braid directly affects the suture retention strength. This result was expected, as PGA has higher tensile strength compared to PLA.

### Example 2

### In vitro degradation study

Fascia discs having a diameter of 4 cm were stitched along the periphery using PLA, PGA, and PE polymer braids (n=6 per group). Three specimens per group were allocated to time zero testing and three were subjected to *in vitro* degradation. For *in vitro* degradation, the specimens were put in individual beakers containing 100 mL of 1xPBS (pH=7.4) and immersed in a water bath maintained at 37°C.

The 1xPBS solution was checked every day for any signs of contamination and the solution was changed every other day so as to maintain a constant pH of 7.4 throughout the study. At the end of the 21 days the specimens were removed and sutured to a stainless steel ring in simple suture configuration at 1 cm intervals. The suture retention loads of the two groups, time zero and 21 days, were quantified using the modified ball burst test. Failure testing included 10 cycles of preconditioning form 5-15 N at .25 Hz followed by load to failure at 30 mm/min.

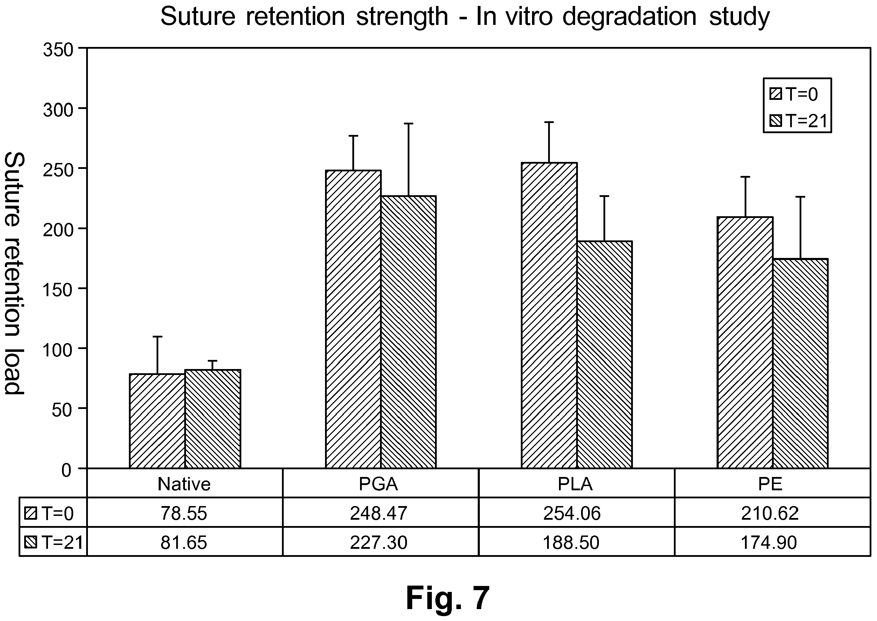

Suture retention load of fascia stitched with the three polymer braids at the two time points, time zero and 21 days, is shown in Fig. 7. Fig. 7 shows that the suture retention loads for fascia stitched with the three polymer braids is significantly higher than native fascia at both time zero and 21 days. The suture retention load of stitched fascia was not significantly different within a group as a function of time, or between groups at either time point. The data show that stitched fascia significantly increases the suture retention load of fascia and the increase is maintained for at least 21 days in simulated *in vivo* conditions.

### Example 3

### Design configurations

4 x 4 cm pieces of fascia were stitched using 2-0 commercial silk suture (Harvard Apparatus, Holliston, MA) using five stitch configurations: 1) peripheral double pass; 2) 2 rectangle double pass; 3) 3 rectangle double pass; 4) 4 rectangle double pass; and 5) rectangular cross-hatch. The samples were tested using the previously described modified ball burst test and a pseudo side constraint test. For the pseudo side constraint test, the sample was constrained to a stainless steel ring using simple suture configurations and was distracted in uniaxial tension to failure at 30 mm/min.

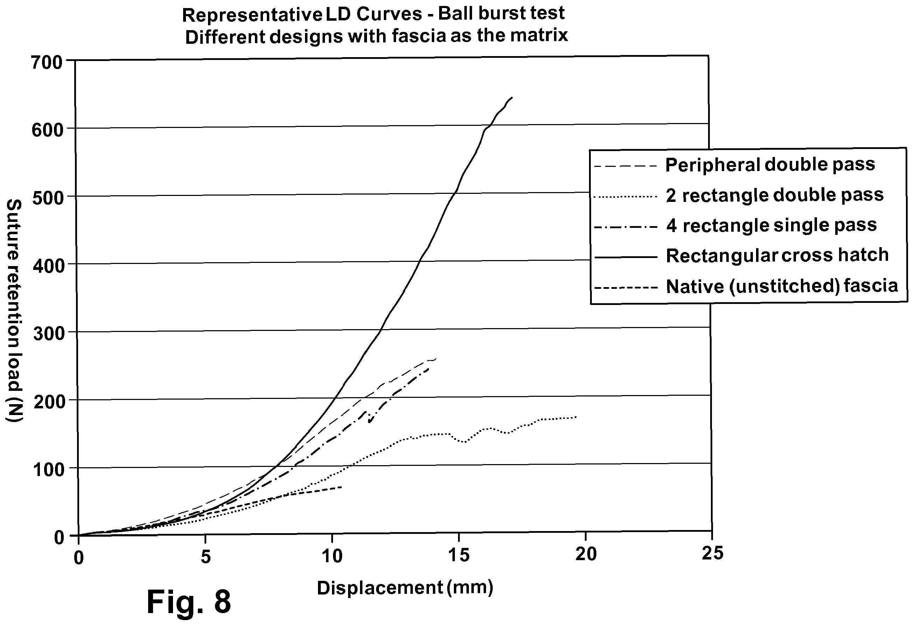

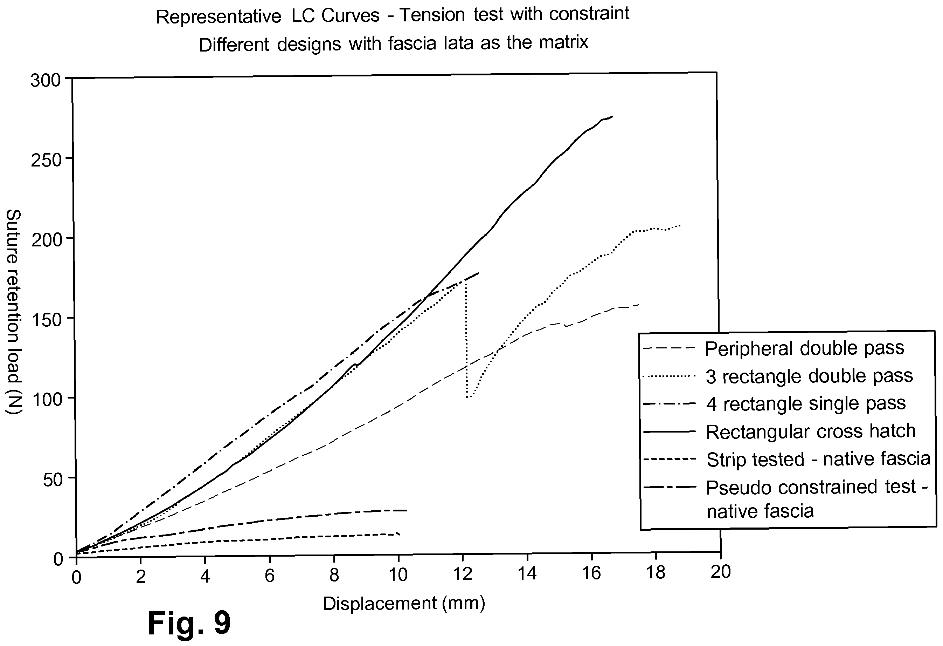

The results are illustrated in Figs. 8-9. Using both test methods, the rectangular cross-hatch stitch pattern had the highest suture retention loads compared to other stitch configurations investigated. These data show that the rectangular cross-hatch stitch pattern will make the mechanical performance of fascia suitable for large to massive rotator cuff tendon repairs.

### Example 4

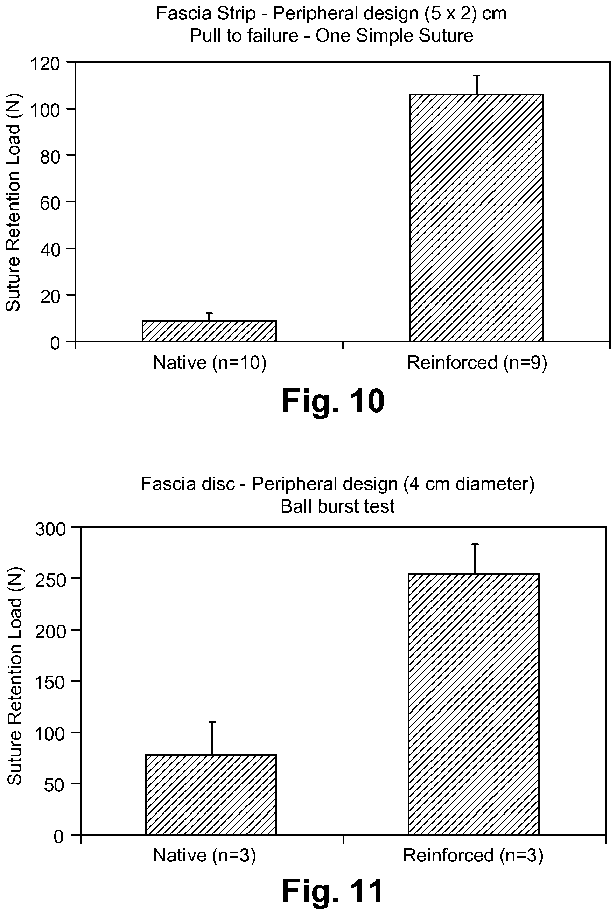

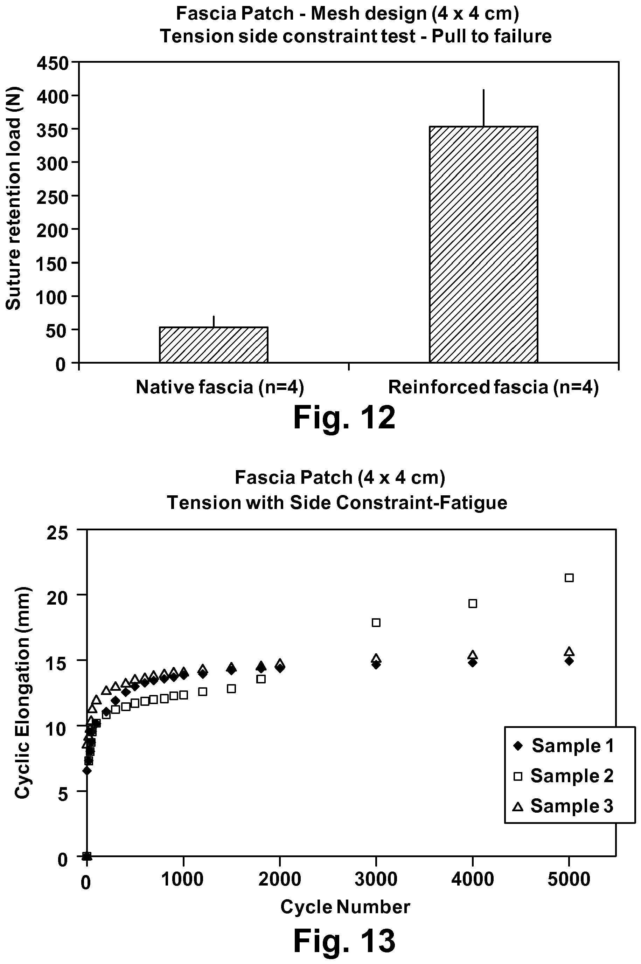

A polymer braid having a 6PLA sheath with 2PGA core (Concordia Medical, Coventry, RI) was stitched into strips and patches of human fascia lata ECM. To model *in vivo* physiologic loading, the suture retention loads were quantified using three different tests: unidirectional pull (Fig. 10), modified ball burst (Fig. 11), and tension with side constraint (Figs. 12-13). For all tests, load was applied to the samples using #2 Fiberwire simple sutures (Arthrex Corporation, Naples, FL).

Specimens were subjected to failure testing using all three types of tests. Failure testing included 10 cycles of pre-conditioning from 5-15N at .25Hz followed by constant rate distraction to failure at 30 mm/min. As well, samples were subjected to cyclic fatigue testing (5-150 N, 5000 cycles at 0.25 Hz in a saline bath) using the tension with side constraint test.

The results indicate that reinforcing fascia lata ECM with a biodegradable polymer significantly increases its suture retention strength to physiologically relevant loads (>100 N) (Figs. 10-12). Further, the reinforced patch can resist cyclic fatigue loading at physiologically relevant loads (5-150 N) for up to 5000 cycles (Fig. 13). Hence, reinforced fascia may provide a natural, strong, and mechanically robust scaffold for bridging tendon or muscle defects.

### Example 5

### Bone fixation method

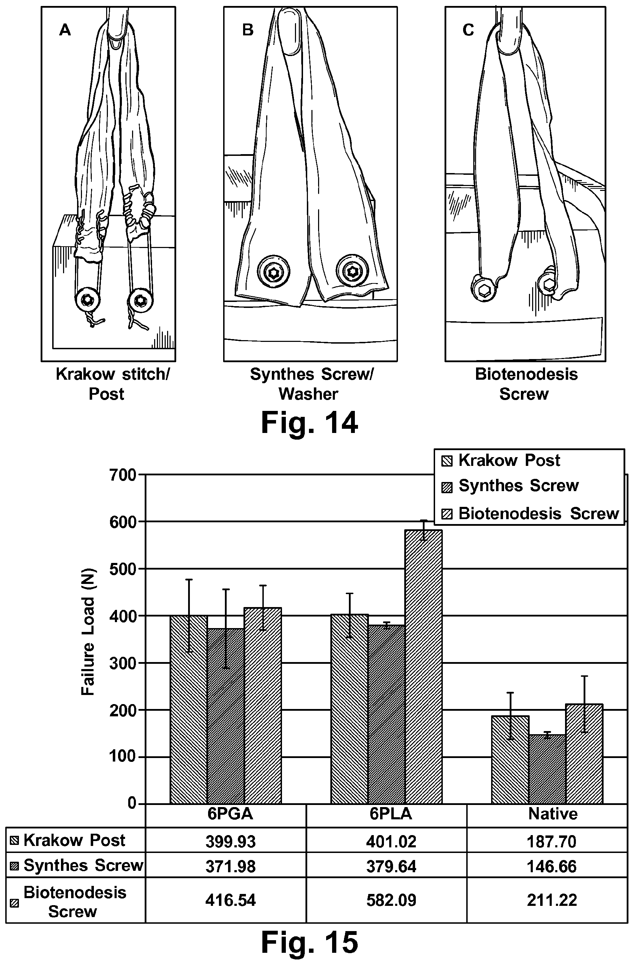

16 x 1.5 cm pieces of fascia were stitched using a peripheral double pass configuration using 6PGA and 6PLA polymer braids. Reinforced fascia was repaired to Sawbones using the following fixation techniques: 1) Krakow stitch with post (models suture anchor) (Fig. 14A, n=2); 2) Screw and washer fixation (Fig. 14B, n=2); and Biotenodesis interference screw (Fig. 14C, n=2). The samples were tested 100 cycles, 5-50 N at .25 Hz and then loaded to failure at 30 mm/min.

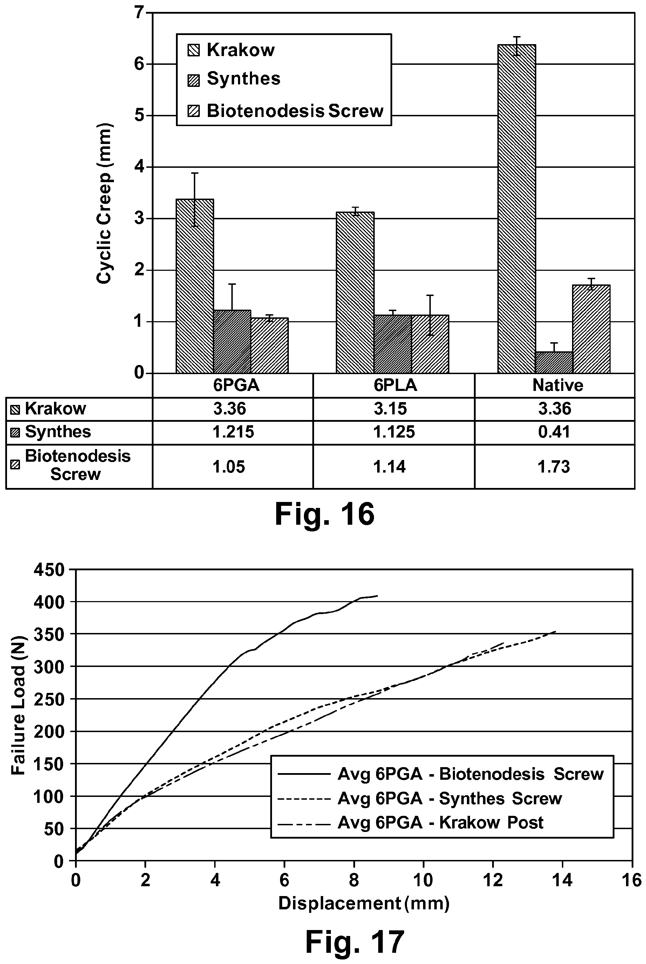

The results are summarized in Figs. 15-17. Failure load is higher in reinforced fascia than native fascia with all bone fixation methods (Fig 15), and failure load is not different between fixation methods or fiber types tested (Fig. 15). Krakow stitch fixation allows more cyclic creep than the other methods (Fig. 16). Representative load-displacement curves for the failure portion of the test for samples reinforced with PGA fiber are shown in Fig. 17. Biotenodesis screw fixation proved to be the stiffest of the three methods during the failure portion of the test (Fig. 17).

### Example 6

### Suture retention test with non resorbable fibers

The studies were conducted to compare the suture retention load of fascia reinforced with non-resorbable polyesters, sizes 2-0 and 3-0 polyethylene terephthalate (PET) braided suture (Ashaway Twin Mfg. Co., RI) and ForceFiber™ UHMWPE braided suture (Teleflex Medical, MA). Suture retention loads were quantified using the uniaxial suture retention test and compared to fascia reinforced with size 2-0 custom braided PLA suture (Concordia Fibers, RI).

Each 2 x 5 cm strip was provided with an inner stitch line placed 5 mm away from the edge of the tissue and an outer stitch line placed 3 mm from the edge of the tissue. A template was created to assure uniformity in the placement of the sutures.

The suture retention load, defined as the maximum load attained by the specimen was quantified using a standard pull to failure test with one simple suture. The specimen was preloaded to 2 N and then distracted to failure at a rate of 30 mm/min.

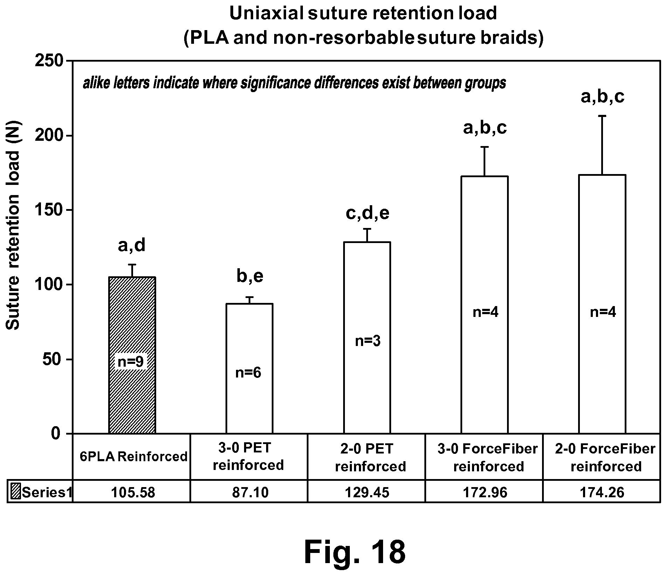

The results of this test are illustrated in Fig. 18. Fig. 18 shows that stitching as a reinforcement method has the ability to increase the suture retention strength of fascia (compare to unreinforced fascia shown in Fig 10). Even though different polymer braids have been used, the table clearly indicates an increase in suture retention properties, irrespective of the polymer braid used as the reinforcing material. Additionally, suture retention strength of the fascia reinforced with ForceFiber in both sizes was significantly higher compared to fascia reinforced with the PET braided and PLA braided suture materials. No significant difference, however, in suture retention strength was found between 2-0 (174 ±39 N) and 3-0 (173 ± 20 N) ForceFiber reinforced fascia. Further, the 2-0 PET reinforced fascia (129 ± 8 N) had a significantly higher suture retention load compared to the 3-0 PET reinforced fascia (87 ± 5 N) and the PLA reinforced fascia (106 ± 9 N).

### Suture Displacement Test

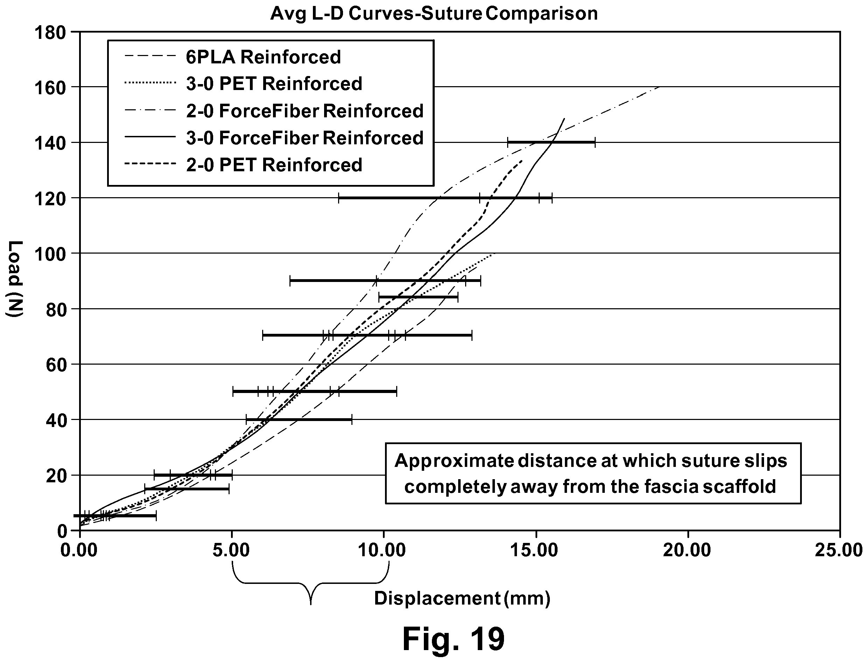

Fig. 19 gives the average load displacement plots (LD) of 2 x 5 cm strips of fascia reinforced with 2-0 and 3-0 sized PET braided suture (Ashaway Twin Mfg. Co., RI) and ForceFiber UHMWPE braided suture (Teleflex Medical, MA). The LD plot was also generated for fascia reinforced with 6PLA.

The LD plots for fascia reinforced with different suture materials is essentially the same until 5 mm of displacement. Visual inspection suggests that initially both the fibers and fascia matrix are loaded as the fibers slip through the fascia matrix. After about 5 mm of displacement, however, the fibers completely slip out from the fascia matrix and become the primary load bearing components of the reinforced fascia construct.

The complete slippage of fibers from the fascia matrix corresponds to the initial placement of the stitch lines at 5-10 mm from the edge of the fascia patch. After the fiber has completely slipped from the fascia, the maximum load attained by the reinforced fascia construct depends on the ultimate tensile strength and knot breaking strength of the respective fibers used to reinforce the fascia.

For the PET samples, slipping of the fiber at the fiber-fascia interface was followed by breaking of the stitched fiber loop at displacements greater than 5 mm. The ForceFiber samples failed when the inner stitch line unraveled in the direction of loading together with pulling along the stitching lines and breaking of the stitched loops.

It may be concluded from the LD plots that 2-0 ForceFiber reinforced fascia is stiffer than fascia reinforced with other suture materials. The large error bars seen in Fig. 19 for the 2-0 ForceFiber reinforced fascia are due to the divergent behavior of one of the four tested specimens.

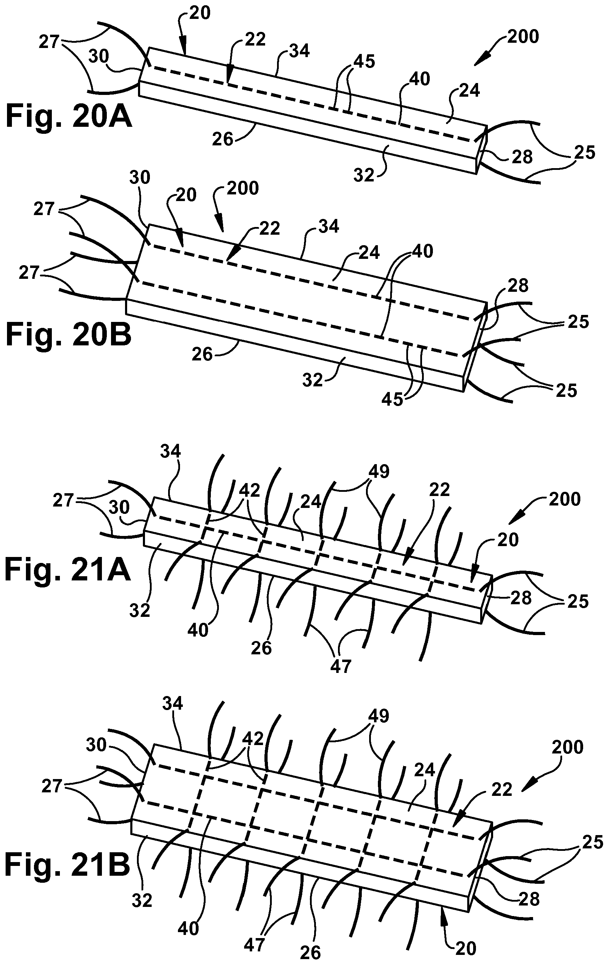

One example of a tissue graft 200 in accordance with yet another embodiment of the present invention is illustrated in Fig. 20A. The tissue graft 200 includes a reinforced ECM strip or patch 20 that can be used to augment a tendon or muscle repair to bone in, for example, a rotator cuff repair. The tissue graft 200 includes an ECM patch 20 and a means 22 for reinforcing the patch. The graft 200 is similar to the graft 10 illustrated in Fig. 1 except that in the graft 200 the reinforcing means 22 includes one or more free end portions that extend through and beyond the periphery of the graft. More specifically, the tissue graft 200 includes one or more surfaces that define the periphery of the tissue graft (*i.e.,* the outer boundaries of the tissue graft). For example, the top surface 24, bottom surface 26, first side 28, second side 30, front surface 32, and rear surface 34 of the patch 20 cooperate to define the periphery of the patch. Accordingly, it will be appreciated that the free ends of the reinforcing means 22 may extend through and beyond any one or combination of surfaces 24-34 defining the periphery of the patch 20 in accordance with the present invention. The periphery of the patch 20 may also include more or fewer surfaces given the shape of the patch. Since each of the surfaces 24-34 includes the edges of the patch 20 that define each surface, the reinforcing means 22 may extend through the edges and/or interior of each surface of the patch in accordance with the present invention.

The reinforcing means 22, constituting one linearly extending first fiber 40 formed from stitches 45, is integrated into the patch 20 such that free end portions 25, 27 of the first fiber extend through and beyond one or more peripheral surfaces 24-34 of the tissue graft 200. In Fig. 20A, two portions 25 of the first fiber 40 extend through the top surface 24 and bottom surface 26, respectively, near the first side 28 of the patch 20 and away from the patch. Likewise, two portions 27 of the first fiber 40 extend through the top surface 24 and bottom surface 26, respectively, near the second side 30 of the patch 20 and away from the patch. The portions 25 and the portions 27 therefore extend beyond opposing peripheral surfaces 28, 30 of the patch 20. The portions 25, 27 are used to secure the tissue graft 200 to the host tissue(s) using conventional fastening techniques. It will be appreciated, however, that more or fewer (including zero) portions 25, 27 of the fiber 40 may extend through the top and bottom surface 24, 26 at each respective side 28, 30 of the patch 20 (not shown).

In Fig. 20B, a pair of spaced-apart first fibers 40 extends linearly along the length of the patch 20. Each fiber 40 includes free end portions 25 that extend through and away from the top and bottom surfaces 24, 26, respectively, near the first side 28 and free end portions 27 that extend through and away from the top and bottom surfaces, respectively, near the second side 30 of the patch 20 of the graft 200. It will be appreciated, however, that more or fewer (including zero) portions 25, 27 of each fiber 40 may extend through the top and bottom surface 24, 26 at each respective side 28, 30 of the patch 20 (not shown).

Referring to Figs. 21A and 21B, the patch 20 of the may further include one or more linearly extending second fibers 42 that extend transverse to and intersect one first fiber 40 (Fig. 21A) or multiple first fibers 40 (Fig. 21B). The second fibers 42 may extend perpendicular to the first fiber(s) 40 or may extend at an acute or obtuse angle relative to the first fiber(s). In any case, the second fibers 42 extend through and away from opposing peripheral surfaces of the patch 20. In particular, each second fiber 42 includes one or more free end portions 47 that extend through the top surface 24 and bottom surface 26, respectively, near the front surface 32 of the patch 20 and one or more free end portions 49 that extend through the top surface and bottom surface, respectively, near the rear surface 34 of the patch 20. The portions 47 and the portions 49 therefore extend beyond opposing peripheral surfaces 32, 34 of the patch 20. It will be appreciated that more or fewer portions 47, 49 of each second fiber 42 may extend through the top and bottom surfaces 24, 26 at each respective surface 32, 34 of the patch 20 (not shown).

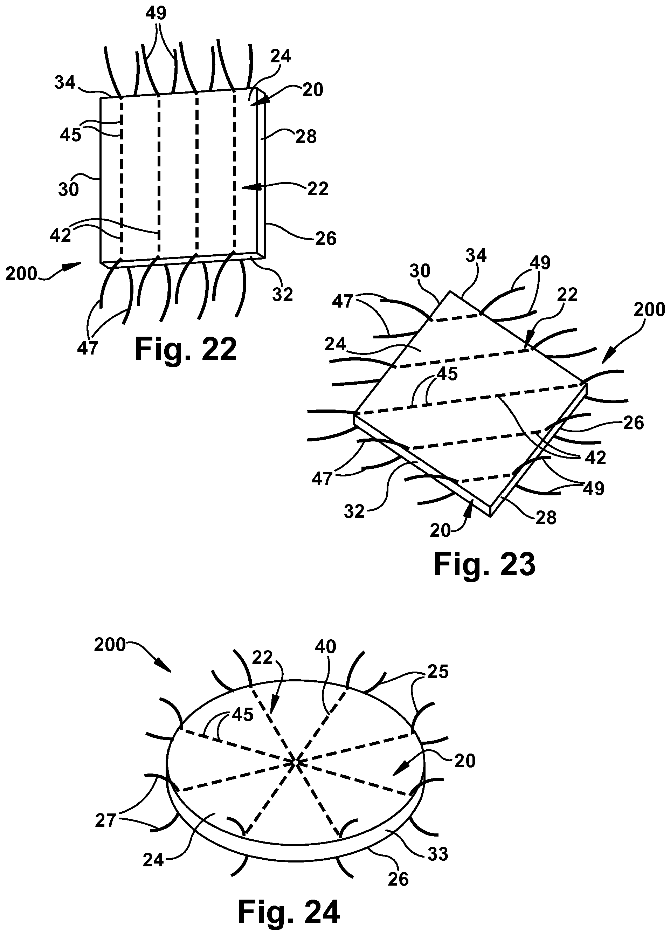

Referring to Figs. 22 and 23, the reinforcing means 22 includes a plurality of linearly extending second fibers 42 while the first fiber(s) 40 are omitted. Each second fiber 42 includes portions 47 that extend through the top surface 24 and bottom surface 26, respectively, near the front surface 32 and portions 49 that extend through the top and bottom surface near the rear surface 34. In Fig. 23, the second strands 42 extend transversely across the tissue graft 200 such that the portions 47, 49 of each second strand extend beyond ends or sides of the patch 20 that do not oppose one another, *e.g*., the ends or sides are adjacent to one another. In other words, the portions 47, 49 of the second strands 42 do not extend solely through the top and bottom surfaces 24, 26 at the opposing front and rear surfaces 32, 34. Instead, the first portions 47 of the second strands 42 extend through the top and bottom surfaces 24, 26 at a non-perpendicular angle relative to both the front surface 32 and the adjacent second side 30 of the patch 20. Likewise, the second portions 49 of the second strands 42 extend through the top and bottom surfaces 24, 26 at a non-perpendicular angle relative to both the rear surface 34 and the adjacent first side 28 of the patch 20. It will be appreciated that more or fewer portions 47, 49 of each second fiber 42 may extend through the top and bottom surfaces 24, 26 near respective surfaces 30, 32 and 28, 34 of the patch 20 (not shown).

Although the patches 20 of the grafts 200 in Figs. 20-23 are illustrated as being rectangular or square, it will be appreciated that the graft could alternatively be, for example, circular as shown in Fig. 24. In such a case, the patch 20 has an arcuate or circular peripheral surface 28. Each first fiber 40 has a linear configuration and extends through the top and bottom surfaces 24, 26 near the peripheral surface 28 at multiple locations. The first fibers 40 may extend through or intersect at the center of the circular patch 20or may be spaced from the center of the circular graft (not shown). Portions 25 of each first fiber 40 extend through the top and bottom surfaces 24, 26 near one side of the surface 33 relative to the center of the patch 20 while portions 27 of each first fiber extend through the top and bottom surfaces near a different side of the surface relative to the center. It will be appreciated that more or fewer portions 25, 27 of each first fiber 40 may extend through the top and bottom surfaces 24, 26 near different sides or portions of the surface 33 of the patch 20 (not shown).

The configuration of the reinforcement means 22 for the graft 200 is not limited to those shown in Figs. 20A-24. The reinforcement means 22 of the graft 200 may, for example, exhibit the concentric or cross-hatched configurations shown in Figs. 1-4B. In these instances, the reinforcement means 22 still includes at least one free end that extends through and beyond at least one peripheral surface of the patch 20.

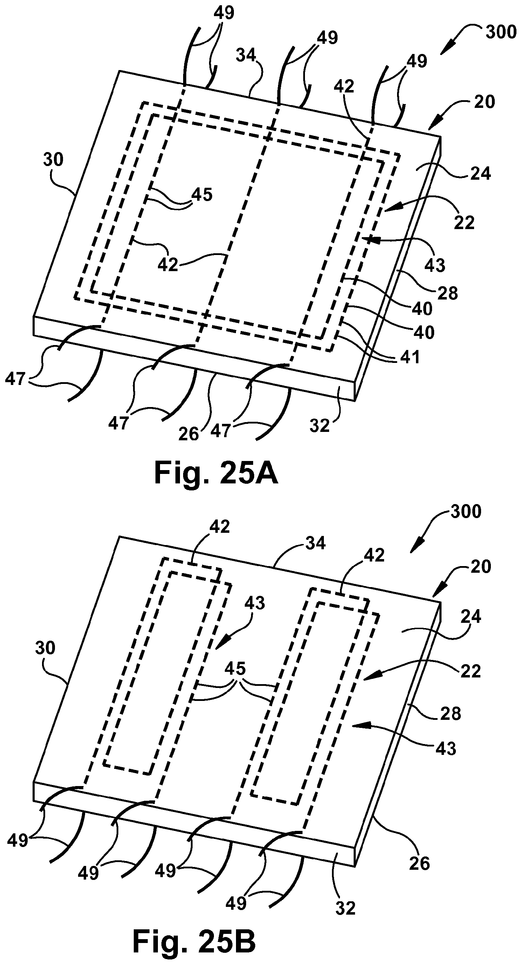

One example of a tissue graft 300 in accordance with yet another embodiment of the present invention is illustrated in Fig. 25A. The tissue graft 300 includes an ECM patch 20 and means 22 for reinforcing the patch. The graft 300 is similar to the grafts 200 illustrated in Figs. 20A-24 except that in the graft 300 the reinforcing means 22 includes both a concentric pattern 43 of first fibers 40 and linearly extending second fibers 42 with one or more free end portions 47, 49 that extend through and beyond the periphery of the graft. The linearly extending second fibers 42 traverse the concentric pattern 43 of first fibers 40 although one or more of the second fibers may alternatively be spaced away from the concentric pattern (not shown). The portions 47, 49 of the second fibers 42 may extend through any combination of peripheral surfaces 24-34 of the patch 20 of the graft 300.

Fig. 25B illustrates another example of reinforcing means 22 comprising two substantially concentric patterns 43 formed by a pair of second fibers 42. More specifically, each second fiber 42 forms two substantially rectangular shapes that overlap one another while both ends of the fiber extend linearly along the patch towards the peripheral surface 32. Both the portions 47 and the portions 49 of each second fiber 42 extend through and beyond the same peripheral surface 32, although the portions may extend through and beyond any combination of peripheral surfaces 24-34 of the patch 20. Although two patterns 43 are illustrated, it will be appreciated that more or fewer patterns may be provided in the patch 20 in accordance with the present invention.

The grafts 300 in Figs. 25A-25B are illustrative and not exhaustive examples of tissue grafts that may include both concentric and linearly extending reinforcement means 22 with free ends extending through and beyond peripheral surfaces of the graft in accordance with the present invention. More specifically, tissue grafts may be provided that include the concentric patterns 43 of Figs. 3A-3B and/or the linearly extending cross-hatched pattern of Figs. 4A-4B.

### Example 7

In this example, the tissue layer is reinforced with fiber in a manner that strengthens and stiffens the tissue layer and is also used for attachment to host tissues.

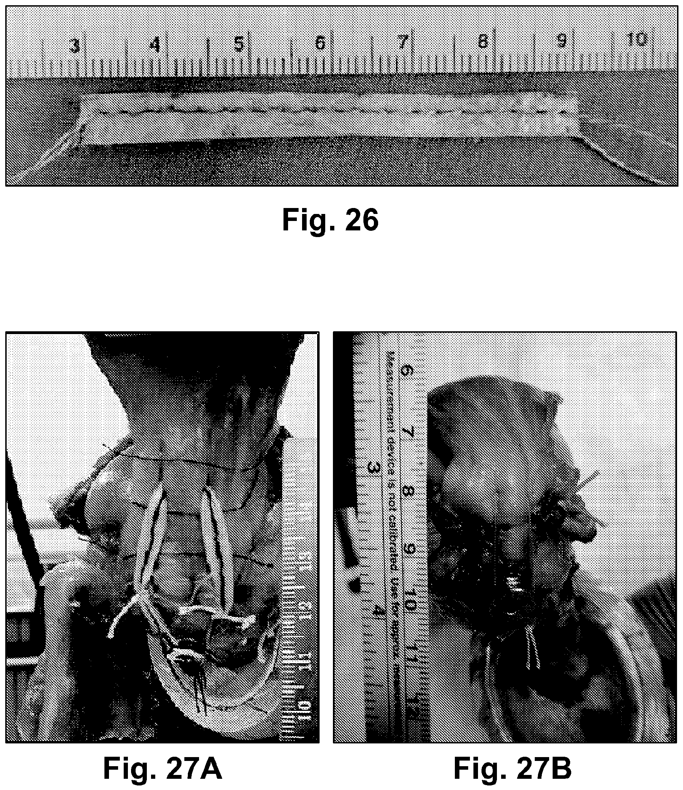

The tissue layer could be, for example, a 0.5 x 6 cm strip of ECM derived from human fascia lata (Musculoskeletal Transplant Foundation, Edison, NJ) and the fiber could be a UHMWPE braid (ForceFiber, TeleFlex Medical OEM, Kenosha, WI) stitched in a single pass across the tissue layer (Fig. 26).

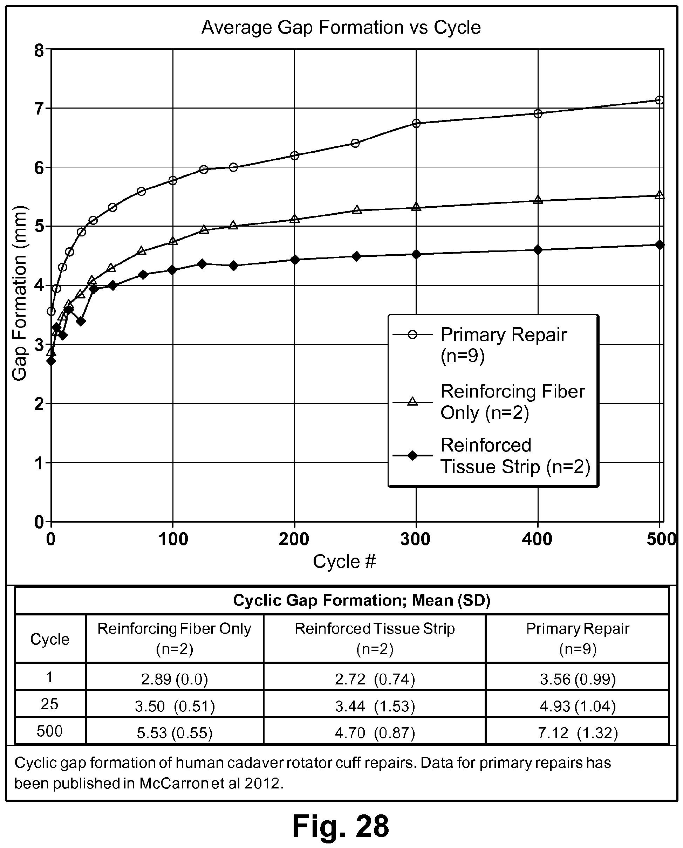

Two pairs of human cadaveric shoulders (mean age, 55 ± 9 years) were used in this study (Anatomy Gifts Registry, Glen Burnie, MD and ScienceCare, Aurora, CO). In each shoulder, the supraspinatus was sharply released from the proximal humerus and primarily repaired back to its insertion with anchors. For each pair of shoulders, one repair was randomly assigned for augmentation with a reinforced fascia strip (Fig. 27A) and the other repair was augmented with the reinforcing fiber alone (Fig. 27B). The strips or fiber alone were passed through the tendon repair approximately 1 cm medial to the repair suture line in a mattress configuration and affixed to the humerus laterally with a bone anchor. Repairs were subjected to cyclic mechanical loading of 5-180N at 0.25Hz for 500 cycles. Repairs were tested in air, at room temperature, and kept moist by intermittent spraying with saline solution. Optical markers were used to monitor gap formation across the repair during cyclic loading. Repair augmentation with either the reinforcing fiber alone or the reinforced tissue strip reduced cyclic gap formation compared to repairs with no augmentation (Fig. 28). Repair augmentation with the reinforced tissue strip reduced cyclic gap formation to a greater extent than augmentation with the reinforcing fiber alone (Fig. 28).

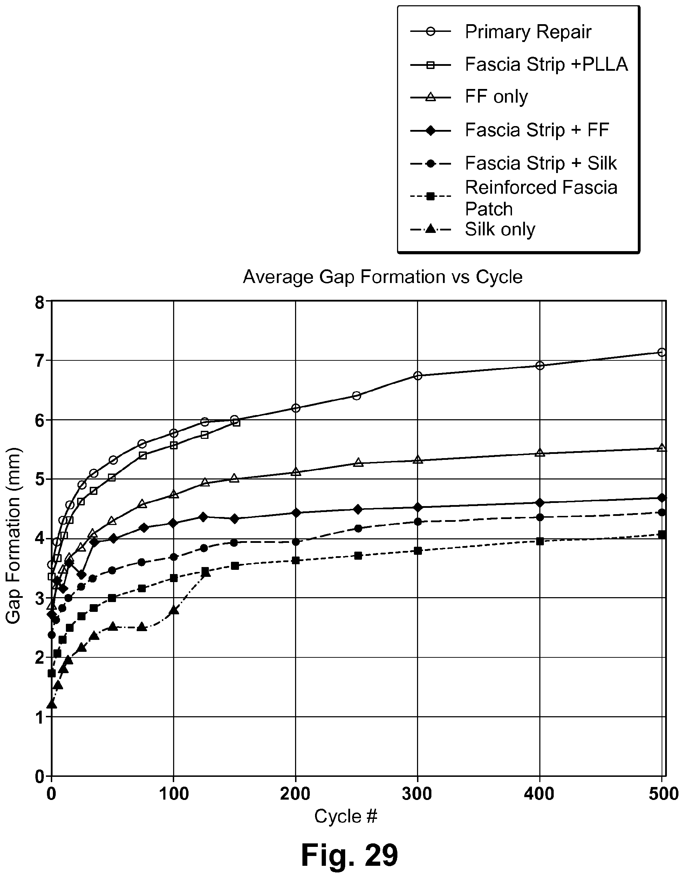

Using a human cadaver model of rotator cuff repair, this example demonstrates how repair augmentation reduces cyclic gap formation and augmentation with a reinforced tissue strip is more mechanically advantageous than augmentation with the reinforcing fiber alone. Therefore, using the reinforced tissue strip, both the tissue and the fiber have a biomechanical role in repair augmentation. Fig. 29 compares the average gap formation for alternative configurations for both the reinforced tissue strip and the reinforcing fiber alone.

### Example 8

In this example, the patch is reinforced with fiber in a manner that limits cyclic stretching of the patch following partial enzymatic degradation of the patch. The patch could be, for example, a 5 x 5 cm patch of ECM derived from human dermis (DermaMatrix, Musculoskeletal Transplant Foundation, Edison, NJ) and the fiber could be a #1 PLA/PGA braid (6PLA sheath with 2PGA core, Concordia Medical, Coventry, RI) stitched in a crosshatch pattern across the patch. In other embodiments, the patch could be an allograft material derived from other tissues, a xenograft material or a synthetic mesh, and the stitching fiber could be derived from other synthetic biomaterials with different biodegradation profiles (such as polypropylene, UHMWPE, ePTFE, PLGA and PLLA) or natural biomaterials (such as silk and collagen).

In this example, an *in vitro* accelerated enzymatic degradation model (graft degradation in collagenase solution (21U/ml) at 37°C for eight hours) was used to simulate in vivo degradation of an ECM graft. Native and reinforced dermis patches were mechanically tested before ("time-zero") and after enzymatic degradation (n=6/group/condition) in a custom ball-burst test setup with the patch fixed using eight peripheral mattress suture loops of #2 FiberWire to the fixture. The patches were preloaded to 10N and then cyclically loaded 10N-80N (the expected physiologic load on a 5x5cm abdominal wall patch) for 1000 cycles. The pretension elongation (PE), cyclic elongation (CE) and cumulative CE (CCE=PE+CE) were measured for each cycle. All data were analyzed by 2-way ANOVA and *post hoc* Tukey tests; p<0.05 was considered to be significant.

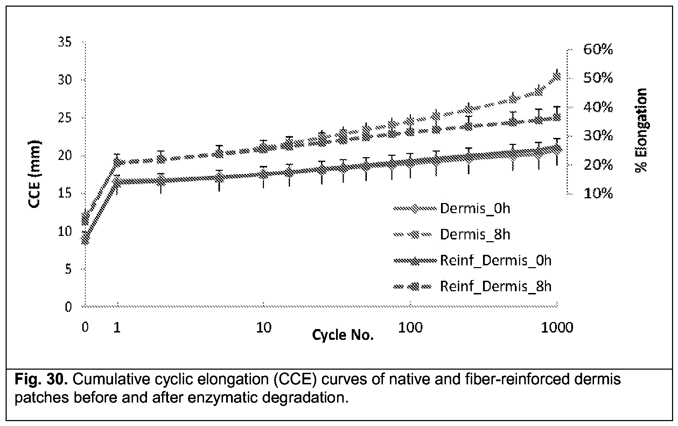

At time-zero, native and reinforced dermis patches underwent similar (∼21mm, p=0.98) and significant (p<0.05) elongation (or stretch) on cyclic loading elongation after 1000 cycles (Fig. 30 and Table 1). Enzymatic degradation resulted in significantly higher rate and amount of elongation (28.4±2.1 mm) in the native dermis group compared to the reinforced group (25.0±1.5 mm; p<0.001). One patch in the enzyme-degraded native dermis group failed during cyclic loading (at cycle 971).

| **Table 1.** Cumulative elongation of native and fiber-reinforced dermis patches after pretensioning and cyclic loading (10-80N, 1000 cycles). Significant differences are indicated by like letters. *One patch in the enzyme-degraded native dermis group failed during cyclic loading (at cycle 971). | | | | |
|---|---|---|---|---|
| | ***Time Zero (0h) (n=6)*** | | ***Collagenase (8h) (n=6)*** | |
| **Elongation (mm)** | **Native Dermis** | **Fiber-reinforced Dermis** | **Native Dermis** | **Fiber-reinforced Dermis** |
| **10N Pretension** | 9.1 ± 0.8 | 8.9 ± 1.0 | 11.9 ± 1.3 | 11.3 ±1.1 |
| **Cycle 1** | 16.6 ± 1.8 | 16.4 ±1.0 | 19.0 ± 1.9 | 19.1 ±1.1 |
| **Cycle 10** | 17.5 ± 1.9 | 17.5 ±1.1 | 21.1 ± 2.2 | 20.8 ±1.2 |
| **Cycle 100** | 19.0 ± 2.0 | 19.3 ±1.2 | 24.5 ± 2.2 | 23.1 ±1.3 |
| **Cycle 1000** | 20.8 ± 2.1^{a} | 21.2 ± 1.2^{b} | 28.4 ± 2.1*^{a,c} | 25.1 ±1.5^{b,c} |

Using an *in vitro* model of enzymatic degradation of ECM grafts, this example demonstrates that dermis patches reinforced with PLLA/PGA fiber limited the cyclic stretching of the grafts after partial enzymatic degradation. Therefore, the presence of reinforcing fiber is expected to limit stretching of the ECM graft after surgical implantation such as in abdominal wall and hernia repairs, and potentially improve repair outcomes by preventing repair bulging and reherniation.

## Claims

1. A biocompatible tissue graft comprising:
an extracellular matrix patch having a peripheral surface; and
at least one fiber stitched in a reinforcing pattern to mitigate tearing of the graft and/or improve the fixation retention of the graft and substantially maintain these properties following partial enzymatic degradation of the graft while it remodels, the at least one fiber having at least one free end extending beyond the peripheral surface of the extracellular matrix for securing the patch to a host tissue.

2. The tissue graft of claim 1, wherein the patch is decellularized.

3. The tissue graft of claim 1, wherein the patch further comprises at least one progenitor cell; or
wherein the patch further comprises at least one biologically active molecule selected from the group consisting of drugs, sclerosing agents, enzymes, hormones, cytokines, colony-stimulating factors, vaccine antigens, antibodies, clotting factors, angiogenesis factors, regulatory proteins, transcription factors, receptors, and structural proteins, nucleic acid therapeutic agents, and combinations thereof.

4. The tissue graft of claim 1, wherein the extracellular matrix patch comprises a fascia patch.

5. A method of constructing a biocompatible tissue graft comprising:
providing an extracellular matrix patch having a peripheral surface;
stitching at least one fiber into the patch in a reinforcement pattern to mitigate tearing of the graft and/or improve the fixation retention of the graft and substantially maintain these properties following partial enzymatic degradation of the graft while it remodels, the at least one fiber having a free end that extends beyond the peripheral surface and being effective for securing the graft to a host tissue.

6. The tissue graft of claim 1 or the method of claim 5, wherein the at least one fiber is stitched into the patch to extend at a non-perpendicular angle to the peripheral surface of the patch.

7. The tissue graft of claim 1 or the method of claim 5, wherein the at least one fiber is stitched into the patch to extend beyond opposing peripheral surfaces of the patch.

8. The tissue graft of claim 1 or the method of claim 5, wherein the at least one fiber comprises a first fiber stitched into the patch to extend beyond a first pair of opposing peripheral surfaces and a second fiber stitched into the patch to extend beyond a second pair of opposing peripheral surfaces different from the first pair.

9. The tissue graft of claim 1 or the method of claim 5, wherein the step of stitching at least one fiber into the patch comprises stitching fibers into the patch in at least one of a concentric pattern, a linear pattern, multiple concentric patterns, and/or a cross-hatched pattern.

10. The tissue graft of claim 1 or the method of claim 5, wherein the fiber comprises a biocompatible material that is bioresorbable, biodegradable, or non-resorbable.

11. The tissue graft of claim 1 or the method of claim 5, further comprising the step of modifying at least one of the fiber or the patch to improve adhesion between the fiber and the patch.

12. The tissue graft of claim 1 or the method of claim 5, wherein the at least one fiber is radio-opaque to allow the location, integrity, and/or deformation of the tissue graft to be assessed in a living system.

13. A tissue graft for use in a method for repairing tissue in a subject comprising:
providing the tissue graft having a peripheral surface and comprising an extracellular matrix patch and at least one fiber stitched into the patch in a reinforcement pattern to mitigate tearing of the graft and/or improve the fixation retention of the graft and substantially maintain these properties following partial enzymatic degradation of the graft while it remodels; and
securing a free end of the at least one fiber that extends beyond the peripheral surface of the tissue graft to the tissue in the subject.

14. The tissue graft of claim 1 or the method of claim 5, wherein the at least one fiber is stitched in a reinforcing pattern to limit cyclic stretching of the graft.

## Patentansprüche

1. Biologisch kompatibles Gewebetransplantat, das Folgendes umfasst:
einen Patch von extrazellulärer Matrix, der eine Umfangsfläche aufweist; und
mindestens eine Faser, die in einem verstärkenden Muster vernäht ist, um das Zerreißen des Transplantats zu minimieren und/oder die Beibehaltung der Befestigung des Transplantats zu verbessern und im Wesentlichen diese Eigenschaften nach teilweisem enzymatischem Abbau des Transplantats, während es remodelliert wird, zu erhalten,
wobei die mindestens eine Faser mindestens ein freies Ende aufweist, das sich über die Umfangsfläche der extrazellulären Matrix hinaus zur Sicherung des Patches an einem Wirtsgewebe erstreckt.

2. Gewebetransplantat nach Anspruch 1, wobei der Patch dezellularisiert ist.

3. Gewebetransplantat nach Anspruch 1, wobei der Patch weiter mindestens eine Progenitorzelle umfasst; oder
wobei der Patch weiter mindestens ein biologisch aktives Molekül umfasst, das aus der Gruppe ausgewählt ist, die aus Folgenden besteht: Arzneimitteln, Verödungsmitteln, Enzymen, Hormonen, Zytokinen, koloniestimulierenden Faktoren, Impfstoffantigenen, Antikörpern, Gerinnungsfaktoren, Angiogenesefaktoren, regulatorischen Proteinen, Transkriptionsfaktoren, Rezeptoren und Strukturproteinen, Nukleinsäure-Therapeutika und Kombinationen davon.

4. Gewebetransplantat nach Anspruch 1, wobei der Patch von extrazellulärer Matrix einen Faszien-Patch umfasst.

5. Verfahren zum Aufbau eines biologisch kompatiblen Gewebetransplantats, das Folgendes umfasst:
Bereitstellen eines Patches von extrazellulärer Matrix, der eine Umfangsfläche aufweist;
Vernähen von mindestens einer Faser in dem Patch in einem Verstärkungsmuster, um das Zerreißen des Transplantats zu minimieren und/oder die Beibehaltung der Befestigung des Transplantats zu verbessern und im Wesentlichen diese Eigenschaften nach teilweisem enzymatischem Abbau des Transplantats, während es remodelliert wird, zu erhalten, wobei die mindestens eine Faser ein freies Ende aufweist, das sich über die Umfangsfläche hinaus erstreckt und zur Sicherung des Transplantats an einem Wirtsgewebe wirksam ist.

6. Gewebetransplantat nach Anspruch 1 oder das Verfahren nach Anspruch 5, wobei die mindestens eine Faser in dem Patch vernäht ist, um sich in einem nichtsenkrechten Winkel zur Umfangsfläche des Patches zu erstrecken.

7. Gewebetransplantat nach Anspruch 1 oder das Verfahren nach Anspruch 5, wobei die mindestens eine Faser in dem Patch vernäht ist, um sich über gegenüberliegende Umfangsflächen des Patches hinaus zu erstrecken.

8. Gewebetransplantat nach Anspruch 1 oder das Verfahren nach Anspruch 5, wobei die mindestens eine Faser Folgendes umfasst: eine erste Faser, die in dem Patch vernäht ist, um sich über ein erstes Paar von gegenüberliegenden Umfangsflächen hinaus zu erstrecken, und eine zweite Faser, die in dem Patch vernäht ist, um sich über ein zweites Paar von gegenüberliegenden Umfangsflächen, das sich von dem ersten Paar unterscheidet, hinaus zu erstrecken.

9. Gewebetransplantat nach Anspruch 1 oder das Verfahren nach Anspruch 5, wobei der Schritt des Vernähens von mindestens einer Faser in dem Patch das Vernähen von Fasern in dem Patch in mindestens einem von einem konzentrischen Muster, einem linearen Muster, mehrfachen konzentrischen Mustern und/oder einem Gittermuster umfasst.

10. Gewebetransplantat nach Anspruch 1 oder das Verfahren nach Anspruch 5, wobei die Faser ein biologisch kompatibles Material umfasst, das biologisch resorbierbar, biologisch abbaubar oder nicht resorbierbar ist.

11. Gewebetransplantat nach Anspruch 1 oder das Verfahren nach Anspruch 5, das weiter den Schritt des Modifizierens von mindestens eines der Faser oder dem Patch umfasst, um die Haftung zwischen der Faser und dem Patch zu verbessern.

12. Gewebetransplantat nach Anspruch 1 oder das Verfahren nach Anspruch 5, wobei die mindestens eine Faser strahlungsundurchlässig ist, um zu ermöglichen, dass der Ort, die Unversehrtheit und/oder Verformung des Gewebetransplantats in einem lebenden System bewertet werden kann.

13. Gewebetransplantat für die Verwendung in einem Verfahren für die Reparatur von Gewebe bei einem Subjekt, das Folgendes umfasst:
Bereitstellen des Gewebetransplantats, das eine Umfangsfläche aufweist und einen Patch von extrazellulärer Matrix und mindestens eine Faser umfasst, die in einem Verstärkungsmuster in dem Patch vernäht ist, um das Zerreißen des Transplantats zu minimieren und/oder die Beibehaltung der Befestigung des Transplantats zu verbessern und im Wesentlichen diese Eigenschaften nach teilweisem enzymatischem Abbau des Transplantats, während es remodelliert wird, zu erhalten; und
Sichern eines freien Endes der mindestens einen Faser, die sich über die Umfangsfläche des Gewebetransplantats hinaus zum Gewebe in dem Subjekt erstreckt.

14. Gewebetransplantat nach Anspruch 1 oder das Verfahren nach Anspruch 5, wobei die mindestens eine Faser in einem verstärkenden Muster vernäht ist, um zyklische Dehnung des Transplantats zu begrenzen.

## Revendications

1. Greffon tissulaire biocompatible comprenant :
un timbre de matrice extracellulaire ayant une surface périphérique ; et
au moins une fibre cousue selon un motif de renforcement pour atténuer le déchirement du greffon et/ou améliorer la rétention de fixation du greffon et maintenir sensiblement ces propriétés suite à la dégradation enzymatique partielle du greffon pendant qu'il se remodèle, ladite au moins une fibre ayant au moins une extrémité libre s'étendant au-delà de la surface périphérique de la matrice extracellulaire pour attacher le timbre sur un tissu hôte.

2. Greffon tissulaire de la revendication 1, dans lequel le timbre est décellularisé.

3. Greffon tissulaire de la revendication 1, dans lequel le timbre comprend en outre au moins une cellule progénitrice ; ou
dans lequel le timbre comprend en outre au moins une molécule active biologiquement sélectionnée dans le groupe constitué de médicaments, d'agents sclérosants, d'enzymes, d'hormones, de cytokines, de facteurs de stimulation de colonies, d'antigènes vaccinaux, d'anticorps, de facteurs de coagulation, de facteurs d'angiogenèse, de protéines régulatrices, de facteurs de transcription, de récepteurs, et de protéines structurelles, d'agents thérapeutiques à base d'acide nucléique et de combinaisons de ceux-ci.

4. Greffon tissulaire de la revendication 1, dans lequel le timbre de matrice extracellulaire comprend un timbre de fascia.

5. Procédé de construction d'un greffon de tissu biocompatible comprenant :
la fourniture d'un timbre de matrice extracellulaire ayant une surface périphérique;
le fait de coudre au moins une fibre dans le timbre selon un motif de renforcement pour atténuer le déchirement du greffon et/ou améliorer la rétention de fixation du greffon et maintenir sensiblement ces propriétés suite à la dégradation enzymatique partielle du greffon pendant qu'il se remodèle, ladite au moins une fibre ayant une extrémité libre qui s'étend au-delà de la surface périphérique et étant efficace pour attacher le greffon à un tissu hôte.

6. Greffon tissulaire de la revendication 1 ou procédé de la revendication 5, dans lequel ladite au moins une fibre est cousue dans le timbre pour s'étendre à un angle non perpendiculaire par rapport à la surface périphérique du timbre.

7. Greffon tissulaire de la revendication 1 ou procédé de la revendication 5, dans lequel ladite au moins une fibre est cousue dans le timbre pour s'étendre au-delà des surfaces périphériques opposées du timbre.

8. Greffon tissulaire de la revendication 1 ou procédé de la revendication 5, dans lequel ladite au moins une fibre comprend une première fibre cousue dans le timbre pour s'étendre au-delà d'une première paire de surfaces périphériques opposées et une deuxième fibre cousue dans le timbre pour s'étendre au-delà d'une deuxième paire de surfaces périphériques opposées différentes de la première paire.

9. Greffon tissulaire de la revendication 1 ou procédé de la revendication 5, dans lequel l'étape consistant à coudre au moins une fibre dans le timbre comprend le fait de coudre des fibres dans le timbre selon au moins un motif concentrique, un motif linéaire, plusieurs motifs concentriques, et/ou un motif hachuré.

10. Greffon tissulaire de la revendication 1 ou procédé de la revendication 5, dans lequel la fibre comprend un matériau biocompatible qui est biorésorbable, biodégradable ou non résorbable.

11. Greffon tissulaire de la revendication 1 ou procédé de la revendication 5, comprenant en outre l'étape consistant à modifier au moins soit la fibre soit le timbre pour améliorer l'adhésion entre la fibre et le timbre.

12. Greffon tissulaire de la revendication 1 ou procédé de la revendication 5, dans lequel ladite au moins une fibre est radio-opaque pour permettre d'évaluer l'emplacement, l'intégrité et/ou la déformation du greffon tissulaire dans un système vivant.

13. Greffon tissulaire destiné à être utilisé dans un procédé de réparation d'un tissu chez un sujet comprenant :
la fourniture du greffon tissulaire ayant une surface périphérique et comprenant un timbre de matrice extracellulaire et au moins une fibre cousue dans le timbre selon un motif de renforcement pour atténuer le déchirement du greffon et/ou améliorer la rétention de fixation du greffon et maintenir sensiblement ces propriétés suite à la dégradation enzymatique partielle du greffon pendant qu'il se remodèle ; et
l'attache d'une extrémité libre de ladite au moins une fibre qui s'étend au-delà de la surface périphérique du greffon tissulaire au tissu chez le sujet.

14. Greffon tissulaire selon la revendication 1 ou procédé de la revendication 5, dans lequel ladite au moins une fibre est cousue selon un motif de renforcement pour limiter l'étirement cyclique du greffon.
